# EUROPEAN PATENT APPLICATION

(11) **EP 1 743 938 A1**
(43) Date of publication of application: **17.01.2007**
(21) Application number: 05015135.6
(22) Date of filing: 12.07.2005
(51) Int. Cl.: C12N 15/10, C12N 15/62, C12N 1/21, C12N 1/06

(54) **Means and methods for displaying polypeptides on cells**

(71) Applicant: NascaCell IP GmbH, 81377 München (DE)
(72) Inventor: Adams, Thorsten, 37083 Göttingen (DE); Kolmar, Harald, 64367 Mühltal (DE)
(74) Representative: Bohmann, Armin K.

(57) **Abstract**

Described is a method for presenting a polypeptide of interest on the surface of cells comprising growing and partially lysing cells which express said polypeptide of interest. Also described are methods for identifying cell clones expressing a polypeptide with a desired property and methods for identifying the nucleotide sequences encoding such a polypeptide.
Furthermore, gram-negative bacterial cells are described which contain genetic information for expressing the outer membrane protein and intimin and optionally for expressing a protein of interest.

SEQUENCE LISTING

<110>  Selecore GmbH

<120>  Means and methods for displaying polypeptides on cells

<130>  L1724 EP S3

<160>  11

<170>  PatentIn version 3.3

<210>  1
<211>  234
<212>  DNA
<213>  phage T5

<400>  1
atgaaaaaat tatttttagc tatggcagtg gtacttcttt cggcttgctc tacttttgga      60

cctaaagata ttaaatgtga ggcatactat atgcaggatc atgtgaaata taaggctaat     120

gtttttgata ggaaagggga tatgtttttg gtgtcaccaa ttatggccta tggatctttc     180

tgggcccccg taagctactt taccgaagga aatacatgcg agggagtttt ctaa           234

<210>  2
<211>  77
<212>  PRT
<213>  phage T5

<400>  2

Met Lys Lys Leu Phe Leu Ala Met Ala Val Val Leu Leu Ser Ala Cys
1               5                   10                  15

Ser Thr Phe Gly Pro Lys Asp Ile Lys Cys Glu Ala Tyr Tyr Met Gln
            20                  25                  30

Asp His Val Lys Tyr Lys Ala Asn Val Phe Asp Arg Lys Gly Asp Met
        35                  40                  45

Phe Leu Val Ser Pro Ile Met Ala Tyr Gly Ser Phe Trp Ala Pro Val
    50                  55                  60

Ser Tyr Phe Thr Glu Gly Asn Thr Cys Glu Gly Val Phe
65                  70                  75

<210>  3
<211>  3106
<212>  DNA
<213>  Escherichia coli

<400>  3
ggaaagataa atccgatcta ttaatataat ttatttctca ttctaactca ttgtggtgga      60

gccataacat gattactcat ggttgttata cccggacccg gcacaagcat aagctaaaaa     120

aaacattgat tatgcttagt gctggtttag gattgttttt ttatgttaat cagaattcat 180
ttgcaaatgg tgaaaattat tttaaattgg gttcggattc aaaactgtta actcatgata 240
gctatcagaa tcgccttttt tatacgttga aaactggtga aactgttgcc gatctttcta 300
aatcgcaaga tattaattta tcgacgattt ggtcgttgaa taagcattta tacagttctg 360
aaagcgaaat gatgaaggcc gcgcctggtc agcagatcat tttgccactc aaaaaacttc 420
cctttgaata cagtgcacta ccacttttag gttcggcacc tcttgttgct gcaggtggtg 480
ttgctggtca cacgaataaa ctgactaaaa tgtccccgga cgtgaccaaa agcaacatga 540
ccgatgacaa ggcattaaat tatgcggcac aacaggcggc gagtctcggt agccagcttc 600
agtcgcgatc tctgaacggc gattacgcga aagataccgc tcttggtatc gctggtaacc 660
aggcttcgtc acagttgcag gcctggttac aacattatgg aacggcagag gttaatctgc 720
agagtggtga taactttgac ggtagttcac tggacttctt attaccgttc tatgattccg 780
aaaaaatgct ggcatttggt caggtcggag cgcgttacat tgactcccgc tttacggcaa 840
atttaggtgc gggtcagcgt tttttccttc ctgcaaacat gttgggctat aacgtcttca 900
ttgatcagga tttttctggt gataataccc gtttaggtat tggtggcgaa tactggcgag 960
actatttcaa aagtagcgtt aacggctatt tccgcatgag gcgctggcat gagtcatacc 1020
ataagaaaga ctatgatgag cgcccagcaa atggcttcga tatccgtttt aatggctatc 1080
taccgtcata tccggcatta ggcgccaagc tgatatatga gcagtattat ggtgataatg 1140
ttgctttgtt taattctgat aagctgcagt cgaatcctgg tgcggcgacc gttggtgtaa 1200
actatactcc gattcctctg gtgacgatgg ggatcgatta ccgtcatggt acgggtaatg 1260
aaaatgatct cctttactca atgcagttcc gttatcagtt tgataaatcg tggtctcagc 1320
aaattgaacc acagtatgtt aacgagttaa gaacattatc aggcagccgt tacgatctgg 1380
ttcagcgtaa taacaatatt attctggagt acaagaagca ggatattctt tctctgaata 1440
ttccgcatga tattaatggt actgaacaca gtacgcagaa gattcagttg atcgttaaga 1500
gcaaatacgg tctggatcgt atcgtctggg atgatagtgc attacgcagt cagggcggtc 1560
agattcagca tagcggaagc caaagcgcac aagactacca ggctattttg cctgcttatg 1620
tgcaaggtgg cagcaatatt tataaagtga cggctcgcgc ctatgaccgt aatggcaata 1680
gctctaacaa tgtacagctt actattaccg ttctgtcgaa tggtcaagtt gtcgaccagg 1740
ttggggtaac ggactttacg gcggataaga cttcggctaa agcggataac gccgatacca 1800
ttacttatac cgcgacggtg aaaaagaatg gggtagctca ggctaatgtc cctgtttcat 1860
ttaatattgt ttcaggaact gcaactcttg gggcaaatag tgccaaaacg gatgctaacg 1920
gtaaggcaac cgtaacgttg aagtcgagta cgccaggaca ggtcgtcgtg tctgctaaaa 1980

ccgcggagat gagttcagca cttaatgcca gtgcggttat atttttgat caaaccaagg 2040

ccagcattac tgagattaag gctgataaga caactgcagt agcaaatggt aaggatgcta 2100

ttaaatatac tgtaaaagtt atgaaaaacg gtcagccagt taataatcaa tccgttacat 2160

tctcaacaaa ctttgggatg ttcaacggta agtctcaaac gcaagcaacc acgggaaatg 2220

atggtcgtgc gacgataaca ctaacttcca gttccgccgg taaagcgact gttagtgcga 2280

cagtcagtga tggggctgag gttaaagcga ctgaggtcac tttttttgat gaactgaaaa 2340

ttgacaacaa ggttgatatt attggtaaca atgtcagagg cgagttgcct aatatttggc 2400

tgcaatatgg tcagtttaaa ctgaaagcaa gcggtggtga tggtacatat tcatggtatt 2460

cagaaaatac cagtatcgcg actgtcgatg catcagggaa agtcactttg aatggtaaag 2520

gcagtgtcgt aattaaagcc acatctggtg ataagcaaac agtaagttac actataaaag 2580

caccgtcgta tatgataaaa gtggataagc aagcctatta tgctgatgct atgtccattt 2640

gcaaaaattt attaccatcc acacagacgg tattgtcaga tatttatgac tcatgggggg 2700

ctgcaaataa atatagccat tatagttcta tgaactcaat aactgcttgg attaaacaga 2760

catctagtga gcagcgttct ggagtatcaa gcacttataa cctaataaca caaaaccctc 2820

ttcctggggt taatgttaat actccaaatg tctatgcggt ttgtgtagaa taattccata 2880

accaccccgg ctaaaatatg tattgtttta gtcggggcat aattatttct tcttaagaaa 2940

taacctctta taatcaaatc tactactggt ctttttatct gcttaatagg tctctttcaa 3000

agagacacat tcacgttttc tcatgtaggt tgatccaacc acgctgtata ccaaagctga 3060

atcacatcaa gcaacaacta tgctcacaac atccacacaa taaaaa 3106

<210> 4
<211> 934
<212> PRT
<213> Escherichia coli

<400> 4

Met Ile Thr His Gly Cys Tyr Thr Arg Thr Arg His Lys His Lys Leu
1               5                   10                  15

Lys Lys Thr Leu Ile Met Leu Ser Ala Gly Leu Gly Leu Phe Phe Tyr
            20                  25                  30

Val Asn Gln Asn Ser Phe Ala Asn Gly Glu Asn Tyr Phe Lys Leu Gly
        35                  40                  45

Ser Asp Ser Lys Leu Leu Thr His Asp Ser Tyr Gln Asn Arg Leu Phe
    50                  55                  60

Tyr Thr Leu Lys Thr Gly Glu Thr Val Ala Asp Leu Ser Lys Ser Gln

65                  70                  75                  80

Asp Ile Asn Leu Ser Thr Ile Trp Ser Leu Asn Lys His Leu Tyr Ser
                85                  90                  95

Ser Glu Ser Glu Met Met Lys Ala Ala Pro Gly Gln Gln Ile Ile Leu
            100                 105                 110

Pro Leu Lys Lys Leu Pro Phe Glu Tyr Ser Ala Leu Pro Leu Leu Gly
        115                 120                 125

Ser Ala Pro Leu Val Ala Ala Gly Gly Val Ala Gly His Thr Asn Lys
    130                 135                 140

Leu Thr Lys Met Ser Pro Asp Val Thr Lys Ser Asn Met Thr Asp Asp
145                 150                 155                 160

Lys Ala Leu Asn Tyr Ala Ala Gln Gln Ala Ala Ser Leu Gly Ser Gln
                165                 170                 175

Leu Gln Ser Arg Ser Leu Asn Gly Asp Tyr Ala Lys Asp Thr Ala Leu
            180                 185                 190

Gly Ile Ala Gly Asn Gln Ala Ser Ser Gln Leu Gln Ala Trp Leu Gln
        195                 200                 205

His Tyr Gly Thr Ala Glu Val Asn Leu Gln Ser Gly Asp Asn Phe Asp
    210                 215                 220

Gly Ser Ser Leu Asp Phe Leu Leu Pro Phe Tyr Asp Ser Glu Lys Met
225                 230                 235                 240

Leu Ala Phe Gly Gln Val Gly Ala Arg Tyr Ile Asp Ser Arg Phe Thr
                245                 250                 255

Ala Asn Leu Gly Ala Gly Gln Arg Phe Phe Leu Pro Ala Asn Met Leu
            260                 265                 270

Gly Tyr Asn Val Phe Ile Asp Gln Asp Phe Ser Gly Asp Asn Thr Arg
        275                 280                 285

Leu Gly Ile Gly Gly Glu Tyr Trp Arg Asp Tyr Phe Lys Ser Ser Val
    290                 295                 300

Asn Gly Tyr Phe Arg Met Arg Arg Trp His Glu Ser Tyr His Lys Lys
305                 310                 315                 320

Asp Tyr Asp Glu Arg Pro Ala Asn Gly Phe Asp Ile Arg Phe Asn Gly
                325                 330                 335

Tyr Leu Pro Ser Tyr Pro Ala Leu Gly Ala Lys Leu Ile Tyr Glu Gln
            340                 345                 350

Tyr Tyr Gly Asp Asn Val Ala Leu Phe Asn Ser Asp Lys Leu Gln Ser
        355                 360                 365

Asn Pro Gly Ala Ala Thr Val Gly Val Asn Tyr Thr Pro Ile Pro Leu
    370                 375                 380

Val Thr Met Gly Ile Asp Tyr Arg His Gly Thr Gly Asn Glu Asn Asp
385                 390                 395                 400

Leu Leu Tyr Ser Met Gln Phe Arg Tyr Gln Phe Asp Lys Ser Trp Ser
                405                 410                 415

Gln Gln Ile Glu Pro Gln Tyr Val Asn Glu Leu Arg Thr Leu Ser Gly
            420                 425                 430

Ser Arg Tyr Asp Leu Val Gln Arg Asn Asn Asn Ile Ile Leu Glu Tyr
        435                 440                 445

Lys Lys Gln Asp Ile Leu Ser Leu Asn Ile Pro His Asp Ile Asn Gly
    450                 455                 460

Thr Glu His Ser Thr Gln Lys Ile Gln Leu Ile Val Lys Ser Lys Tyr
465                 470                 475                 480

Gly Leu Asp Arg Ile Val Trp Asp Asp Ser Ala Leu Arg Ser Gln Gly
                485                 490                 495

Gly Gln Ile Gln His Ser Gly Ser Gln Ser Ala Gln Asp Tyr Gln Ala
            500                 505                 510

Ile Leu Pro Ala Tyr Val Gln Gly Gly Ser Asn Ile Tyr Lys Val Thr
        515                 520                 525

Ala Arg Ala Tyr Asp Arg Asn Gly Asn Ser Ser Asn Asn Val Gln Leu
    530                 535                 540

Thr Ile Thr Val Leu Ser Asn Gly Gln Val Val Asp Gln Val Gly Val
545                 550                 555                 560

Thr Asp Phe Thr Ala Asp Lys Thr Ser Ala Lys Ala Asp Asn Ala Asp
                565                 570                 575

Thr Ile Thr Tyr Thr Ala Thr Val Lys Lys Asn Gly Val Ala Gln Ala
            580                 585                 590

Asn Val Pro Val Ser Phe Asn Ile Val Ser Gly Thr Ala Thr Leu Gly
        595                 600                 605

Ala Asn Ser Ala Lys Thr Asp Ala Asn Gly Lys Ala Thr Val Thr Leu
    610                 615                 620

Lys Ser Ser Thr Pro Gly Gln Val Val Val Ser Ala Lys Thr Ala Glu
625                 630                 635                 640

Met Ser Ser Ala Leu Asn Ala Ser Ala Val Ile Phe Phe Asp Gln Thr
                645                 650                 655

Lys Ala Ser Ile Thr Glu Ile Lys Ala Asp Lys Thr Thr Ala Val Ala
            660                 665                 670

Asn Gly Lys Asp Ala Ile Lys Tyr Thr Val Lys Val Met Lys Asn Gly
        675                 680                 685

Gln Pro Val Asn Asn Gln Ser Val Thr Phe Ser Thr Asn Phe Gly Met
    690                 695                 700

Phe Asn Gly Lys Ser Gln Thr Gln Ala Thr Thr Gly Asn Asp Gly Arg
705                 710                 715                 720

Ala Thr Ile Thr Leu Thr Ser Ser Ser Ala Gly Lys Ala Thr Val Ser
                725                 730                 735

Ala Thr Val Ser Asp Gly Ala Glu Val Lys Ala Thr Glu Val Thr Phe
            740                 745                 750

Phe Asp Glu Leu Lys Ile Asp Asn Lys Val Asp Ile Ile Gly Asn Asn
        755                 760                 765

Val Arg Gly Glu Leu Pro Asn Ile Trp Leu Gln Tyr Gly Gln Phe Lys
    770                 775                 780

Leu Lys Ala Ser Gly Gly Asp Gly Thr Tyr Ser Trp Tyr Ser Glu Asn
785                 790                 795                 800

Thr Ser Ile Ala Thr Val Asp Ala Ser Gly Lys Val Thr Leu Asn Gly
                805                 810                 815

Lys Gly Ser Val Val Ile Lys Ala Thr Ser Gly Asp Lys Gln Thr Val
            820                 825                 830

Ser Tyr Thr Ile Lys Ala Pro Ser Tyr Met Ile Lys Val Asp Lys Gln
        835                 840                 845

Ala Tyr Tyr Ala Asp Ala Met Ser Ile Cys Lys Asn Leu Leu Pro Ser
    850                 855                 860

Thr Gln Thr Val Leu Ser Asp Ile Tyr Asp Ser Trp Gly Ala Ala Asn
865                 870                 875                 880

Lys Tyr Ser His Tyr Ser Ser Met Asn Ser Ile Thr Ala Trp Ile Lys
                885                 890                 895

Gln Thr Ser Ser Glu Gln Arg Ser Gly Val Ser Ser Thr Tyr Asn Leu
            900                 905                 910

Ile Thr Gln Asn Pro Leu Pro Gly Val Asn Val Asn Thr Pro Asn Val
        915                 920                 925

Tyr Ala Val Cys Val Glu
    930

<210>  5
<211>  35
<212>  PRT
<213>  artificial sequence

<220>
<223>  artificial sequence

<400>  5

Glu Val Ser Ala Leu Glu Lys Glu Val Ser Ala Leu Glu Lys Glu Val
1               5                   10                  15

Ser Ala Leu Glu Lys Glu Val Ser Ala Leu Glu Lys Glu Val Ser Ala
            20                  25                  30

Leu Glu Lys
        35

<210>  6
<211>  35
<212>  PRT
<213>  artificial sequence

<220>
<223>  artificial sequence

<400>  6

Lys Val Ser Ala Leu Lys Glu Lys Val Ser Ala Leu Lys Glu Lys Val

1               5                   10                  15

Ser Ala Leu Lys Glu Lys Val Ser Ala Leu Lys Glu Lys Val Ser Ala
            20                  25                  30

Leu Lys Glu
        35

<210>  7
<211>  13
<212>  PRT
<213>  artificial sequence

<220>
<223>  artificial sequence

<400>  7

Gly Ala Pro Val Pro Tyr Pro Asp Pro Leu Glu Pro Arg
1               5                   10

<210>  8
<211>  13
<212>  PRT
<213>  artificial sequence

<220>
<223>  artificial sequence

<400>  8

Asp Gly Ser Leu Gly Asp Ile Glu Pro Tyr Asp Ser Ser
1               5                   10

<210>  9
<211>  10
<212>  PRT
<213>  artificial sequence

<220>
<223>  artificial sequence

<400>  9

Ser Ser Thr Ser Ser Asp Phe Arg Asp Arg
1               5                   10

<210>  10
<211>  64
<212>  PRT
<213>  artificial sequence

<220>
<223>  artificial sequence

<400>  10

Pro Pro Thr Pro Leu Gly Ala Pro Val Pro Tyr Pro Asp Pro Leu Glu
1               5                   10                  15

Pro Arg Ala Ala Ser Gly Pro Gly Glu Val Ser Ala Leu Glu Lys Glu
            20                  25                  30

Val Ser Ala Leu Glu Lys Glu Val Ser Ala Leu Glu Lys Glu Val Ser
        35                  40                  45

Ala Leu Glu Lys Glu Val Ser Ala Leu Glu Lys Gly Gly Cys Gly Ser
    50                  55                  60

<210>  11
<211>  70
<212>  PRT
<213>  artificial sequenc

<220>
<223>  artificial sequence

<400>  11

Ser Leu Ile Lys His Trp Val Asp Asp Gly Ser Leu Gly Asp Ile Glu
1               5                   10                  15

Asp Pro Tyr Asp Ser Ser Pro Pro Thr Pro Pro Gly Lys Val Ser Ala
            20                  25                  30

Leu Lys Glu Lys Val Ser Ala Leu Lys Glu Lys Val Ser Ala Leu Lys
        35                  40                  45

Glu Lys Val Ser Ala Leu Lys Glu Lys Val Ser Ala Leu Lys Glu Gly
    50                  55                  60

Gly Cys Gly Pro Gly Met
65                  70

## Description

The present invention relates to a method for presenting a polypeptide of interest on the surface of cells comprising propagating and partially lysing cells which express said polypeptide of interest and to methods for detecting the presence of a protein of interest on the surface of cells.

The present invention also relates to gram-negative bacterial cells which contain genetic information for expressing the outer membrane protein LIp and intimin and optionally a polypeptide of interest.

In recent years, a plethora of methods to obtain proteins with improved or altered properties has been developed. These methods can be subdivided into two different classes, namely rational protein design and molecular evolution. Whereas in the rational design approach, the alterations in the protein are planned based on structural and functional knowledge of the respective protein, the evolutionary approach is based on the generation of large repertoires of different protein variants and the screening of these repertoires for proteins with the desired activity.

An important aspect of molecular evolution is the coupling of a particular phenotype to the corresponding genotype. Commonly, the protein variant, i.e. the phenotype, is tethered to the surface of an entity that contains the genetic information (the genotype) necessary for the production of the respective protein.

Different in vitro and in vivo methods for molecular evolution have been introduced. The most widely used in vivo methods include the presentation of the desired protein on the surface of bacteriophages ("phage display") (Smith 1985), the surface of bacterial cells (Samuelson, Gunneriusson et al. 2002) and the surface of yeast cells (Kondo and Ueda 2004). Protein-nucleic acid conjugates have been successfully used for in vitro evolutionary approaches (Schaffitzel, Hanes et al. 1999; Griffiths and Tawfik 2003). The advantage of cell-based systems, however, lies in the possibility to use fluorescence activated cell sorting for the isolation of clones with the desired properties, an approach that can not be employed with phage display or in vitro methodologies since the particles used in these methods are too small to be detected by the optics of the cell sorter.

For the generation of large molecular repertoires, the gram-negative bacterium Escherichia coli has emerged as the preferred host because it is most suitable for the breeding and maintenance of repertoires derived from up to 10¹⁰ individual transformants (Adams and Kolmar 2004). The use of other organisms is limited by their lower transformation efficiency and thus the limited repertoire sizes that can be obtained.

In order to be presented on the surface of an E. coli cell, the protein to be displayed, has to pass the bacterial cytoplasmic membrane, the periplasm and the outer membrane. In order to achieve this, the passenger protein is normally fused to a translocator domain residing in the outer membrane and exposing the passenger to the extracellular milieu. Different scaffolds for the presentation of passenger proteins on the surface of E. coli cells have been introduced and include porins (Lang 2000), autotransporters (Klauser, Pohiner et al. 1992), fimbriae (Klemm and Schembri 2000a), ice-nucleation-protein (Jung, Lebeault et al. 1998) and intimin (Wentzel, Christmann et al. 2001). However, the presentation of passenger proteins is limited by their passage through the bacterial membranes. The only systematic study published so far on the passage of selected passenger domains through the outer membrane (Adams, Wentzel et al. 2005) comes to the conclusion that membrane translocation is dependent on the folding state of the respective protein. As a consequence, it can be concluded that not all proteins in a given repertoire are amenable to cell surface exposure. The reason for failure of cell surface display, as shown by Adams et. al (Adams, Wentzel et al. 2005), can be sought in the inability of a protein to pass the outer membrane. Similar problems occur in other cell types, such as other bacterial cells or eukaryotic cells. Accordingly, in order to expose any given protein on a cell surface, e.g. on the bacterial cell surface, a system that circumvents the passage through the outer membrane would be advantageous.

Thus, the technical problem underlying the present invention can be seen in the provision of means and methods allowing the construction of systems for an efficient presentation of a protein of interest on the surface of cells, such as bacterial cells.

This problem has been solved by the provision of the embodiments as recited in the claims.

Thus, in a first aspect the present invention relates to a method for presenting a polypeptide of interest on the surface of cells containing genetic information for expressing said polypeptide characterized in that said polypeptide comprises a part which allows it to bind to the surface of said cells and in that the cells are propagated and partially lysed during or after propagation.

The method according to the invention shows the advantage that any polypeptide of interest can be presented on the surface of cells expressing it regardless of whether the protein could be transported over the cell membrane(s) or secreted by the cells. The rational underlying the method according to the invention is that the cells are only lysed partially, i.e. that the cells or a part of the cells remain physically intact to a degree which allows them to present the polypeptide on their surface and that the polypeptide of interest can reach the exterior and is bound to the surface of the cells due to the presence of a part in said polypeptide of interest which ensures binding on the cell surface thereby allowing a phenotype/genotype correlation.

The cells which can be employed in a method according to the invention are basically all types of cells. They can be eukaryotic or prokaryotic cells. Eukaryotic cells are preferably animal cells, more preferably cells which can be propagated in vitro. Preferred are mammalian cells, e.g. human or murine cells. Examples are NIH/3T3 cells (murine) or HG-261 cells (human). In a preferred embodiment, the eukaryotic cells are cells of established cell lines. In another preferred embodiment the eukaryptic cells are fungal cells, e.g. of the genera Saccharomyces, Schizosaccharomyces or Aspergillus.

In a preferred embodiment the cells employed in a method according to the invention are prokaryotic cells, e.g. gram-positive or gram-negative bacterial cells. Preferably the cells are gram-negative bacteria. The term "gram-negative bacterium" when used in the context of the present invention includes all possible gram-negative bacteria. Preferably, the bacterium belongs to an order selected from the group consisting of Neisseriales, Sphingomonadales, Rhizobiales, Enterobacteriales and Xanthomonadales. More preferably it belongs to a family selected from the group consisting of Neisseriaceae, Alcaligenaceae, Sphingomonadaceae, Rhizobiaceae, Enterobacteriaceae and Xanthomonadaceae. Even more preferably, the bacterium belongs to a genus selected from the group consisting of Neisseria, Alcaligenes, Bordetella, Zymomonas, Agrobacterium, Salmonella, Shigella and Xanthomonas.

In a further preferred embodiment the gram-negative bacterium belongs to the genus Pseudomonas, more preferably to the species P. fluorescens, P. putida, P. alcaligenes or P. oleovorans.

The term "surface of cells" means the surface of the cells presented to the exterior. In the case of animal cells, gram-positive bacterial cells and fungal cells, this is generally the cytoplasmic membrane. In the case of gram-negative bacteria, this is the outer cell membrane.

The term "presenting a polypeptide of interest on the surface of cells" means that the polypeptide is displayed on the surface of the cells. The display is achieved by the binding of the polypeptide of interest to the surface of the cells. This binding may be any possible binding based on covalent binding or non-covalent interaction, e.g. ionic interaction, van der Wals bonds, protein-protein interaction, protein-lipid interaction, protein-carbohydrate interaction etc.

The binding of the polypeptide of interest is ensured by equipping the polypeptide of interest with a part (sometimes also referred to as "tag") which is capable to bind to the surface of the cells. This is achieved by making sure that the genetic information contained in the cells which encodes the polypeptide of interest comprises a nucleotide sequence encoding such a part. Such a part may be any kind of physical entity which by way of interaction can confer the capacity of binding to the surface of the employed cells. The target for the binding of the part in the polypeptide of interest to the cell surface may be any kind of molecule or structure presented on the surface of the cells, such as lipids, proteins, carbohydrates, polysaccharides or lipopolysaccharides. Examples and preferred embodiments for the part comprised in the polypeptide of interest are described and explained further below in the context of the cells according to the invention. All the explanations given there also apply here in connection with the method according to the invention.

The cells employed in a method according to the invention contain genetic information for expressing said polypeptide of interest. The term "containing genetic information for expressing" means that the cells contain a nucleotide sequence, typically in the form of DNA, which may be incorporated into the genome or not, which encodes said polypeptide of interest and which is under the control of regulatory elements which allow expression of the polypeptide of interest in the cells employed in the method according to the invention. Such regulatory elements are exhaustively described in the literature for all possible types of cells and are well-known to the person skilled in the art.
The meaning of the term "polypeptide of interest" is described and explained further below in connection with the cells according to the invention. The same explanations as provided there also apply here in connection with the method according to the invention.

The term "propagating" means growing the cells under conditions allowing cell division and cell growth. Means and methods for propagating different types of cells are exhaustively described in the literature and are well-known to the person skilled in the art.

The term "partially lysed" or "partially lysing" means that:
(i) The individual cells of a population of cells are partially lysed in the sense that their membranes are partially lysed and made permeable for the polypeptide of interest. Thus, the cells as such remain basically intact but their membranes are partially lysed. This affects basically all cells of a population of cells. This first meaning of "partially lysed" will be referred to in the following as "incomplete lysis of cells in a population of cells".
(ii) In the alternative, "partially lysed" when referring to cells in a population of cells means that only a part of the cells of the population is lysed whereas the remaining cells are unaffected and remain intact. This second meaning of "partially lysed" will be referred to in the following as "partial lysis of the cells of a population of cells".

"Incomplete lysis" (see (i), above) means that the membranes of the cells are partially lysed. The cells still stay intact and viable, however, their membranes are permeable and allow a polypeptide of interest to reach the exterior of the cells and to bind to the surface of the cells.
Means and methods for achieving incomplete lysis of cells in a population of cells are described in the art. One example is the expression of a construct comprising the membrane-penetrating parts of the E- and L-proteins which are linked by a flexible linker as described in US patent 5,075,223. The degree of incomplete lysis can, e.g., be evaluated by determining the concentration of a reference protein in a culture medium in which the cells are cultivated. By comparing this value with the amount of protein present in an extract of all cells of a population having the same number of cells, one can determine to what extend the reference protein could leave the cells. An example for a reference protein is ribose-binding protein.

"Partial lysis of the cells of a population of cells" means that only a part of the cells of a population of cells is lysed while the remaining cells stay intact. In this alternative, the polypeptide of interest which is set free from lysed cells can bind to the surface of remaining intact cells of said population which also carry the genetic information for expressing the polypeptide of interest thereby allowing a phenotype/genotype correlation.
"Partial lysis" means that at least 5%, preferably at least 10%, more preferably at least 20%, even more preferably at least 30%, particularly preferred at least 40% and most preferably at least 50% of the cells are lysed. The term "partial lysis" moreover means that not more than 95%, preferably not more than 90%, more preferably not more than 80%, even more preferred not more than 70%, particularly preferred not more than 60% and most preferred not more than 50% of the cells of the population are lysed. The degree of partial lysis can, e.g., be determined by measuring the amount of DNA in the culture medium, for example by staining with a fluorescent dye (e.g. CYBR Green; Molecular Probes). Since the amount of DNA is known for a particular organisms, the measured amount of DNA allows to conclude how many cells have been lysed.
"A population of cells" means a multitude of cells, i.e. two or more cells, preferably at least 10², 10³, 10⁴, 10⁵, 10⁶, 10⁷, 10⁸, 10⁹, 10¹⁰, 10¹¹, 10¹², 10¹³, 10¹⁴ or 10¹⁵ cells.

Partial lysis of the cells in a population of cells can be achieved by different approaches.

One possibility which could be used, in particular for eukaryotic cells but also for prokaryotic cells, is the exposure to ultrasonic waves with the energy applied in such a manner that not all but only a part of the cells are lysed. Further possibilities are the addition of detergents, such as SDS, Triton X-100 or Tween-20, in sublethal dosages or the addition of toxic agents, such as Tamoxifen.

In eukaryotic cells partial lysis of the cells of a population of cells can also be achieved by the induction of apoptosis. Addition of certain cytokines, such as INF-γ, induces the expression of caspase via the STAT pathway (Fu, Cell Death Differ. 6 (1999), 1201-1208). The addition of the cytokine at low levels can lead to the induction of apoptosis only in a part of the cells of the population.

Partial lysis of the cells of a population of cells can also be achieved by the expression of proteins in the cells which lead to the lysis of the cells, i.e. lytic proteins. Partial lysis can be ensured by placing the gene encoding the lytic protein under the control of a regulable promoter. The repressor protein which regulates this promoter can then be placed in trans on a temperature-sensitive plasmid. When incubating the cells at a non-permissive temperature, the plasmid, and therefore also the repressor, would be lost by part of the cells of the population. These cells will then get lysed due to the expression of the lytic protein.

Examples for lytic proteins which are suitable for the lysis of bacterial cells are the lysozyme of the white of egg, the E-protein of the phage ΦX174 (Blasi et al., J. Gen. Microbiol. 131 (1985), 1107-1114; Schuller et al., Nucl. Acids Res. 13 (1985), 4143-4153), the L-protein of the phage MS2 (Beremand and Blumenthal, Cell 18 (1979), 257-266), the holin T (Ramanculov and Young, Gene 265 (2001), 25-36), the endolysin E of the phage T4 (Kasai et al., J. Mol. Biol. 34 (1968), 709-712) and the NucE protein of Serratia marcescens (Berkmen et al., J. Bacteriol. 179 (1997), 6522-6524). Expression of such lytic proteins is in particular suitable to obtain partial lysis in populations of bacterial cells. Another possibility to obtain partial lysis is the expression of antimicrobial peptides (Bulet et al., Immunol. Rev. 198 (2004), 169-184) in a subset of the cells. Antimicrobial peptides are cationic peptides with a net charge of +2 to +7 which often show an alpha-helical structure and which are often amphiphatic. These peptide can attack the membranes of cells, in particular bacterial cells (Boman, J. Intern. Med. 254 (2003), 197-215), and lead to lysis. A multitude of antimicrobial peptides are described (more than 1000) which include, e.g., protegrin-1, bactenecin, alpha-defensin and β-defensin (Bulet et al., Immunol. Rev. 198 (2004), 169-184).
Antimicrobial peptides can not only be used to achieve partial lysis of bacterial cells but also of eukaryotic cells. It is known, for example, that ponericin G1, temporin A, tachyplesin-1 and esculentin-1 act haemolytically. Thanatin, tachyplesin-I, brevinin-1, protegrin-1 and misgurin lead to a lysis of yeast cells (Bulet et al., loc. cit.).
The degree of partial lysis in a cell population can be determined, e.g., by comparing the degree of lysis in a population of cells which is treated or manipulated as described above and a control population of cells which have not been treated or manipulated, e.g. by counting the number of cells after the treatment or after a given number of cell cycles or by determining and comparing the growth curves.
The term "partially lysed" preferably means that the degree of lysis is such that the binding of the polypeptide of interest on the surface of cells can be detected, e.g. by a corresponding antibody or ligand.
As described above, partial lysis of cells can be achieved by treating cells with certain physical (e.g. sonification) or chemical (e.g. toxins, detergents) means or by expressing certain (poly)peptides in the cell, preferably in an inducible or regulable manner, which upon a certain stimulus (e.g. chemical or temperature induction) leads to expression of the (poly)peptide and consequently to the lysis of a part of the cells of a population. In another preferred embodiment, the cells employed in a method according to the invention are gram-negative bacteria according to the invention which are described in more detail further below and which contain genetic information for expressing a polypeptide of interest which is capable of binding to the surface of said cells and which contain genetic information for expressing the outer membrane protein LIp and an intimin. It has been found that the coexpression of the outer membrane protein LIp and of intimin in gram-negative bacteria has the effect that the bacterial cells become physically unstable such that a fraction, but not all, of the cells get lysed and release the cellular content. Thus, the polypeptide of interest is released from the cells and can bind to the surface of remaining intact cells of the population thereby allowing a phenotype/genotype correlation. Thus, in a preferred embodiment that present invention relates to a method for presenting a polypeptide on the surface of gram-negative bacteria, in particular on the outer membrane of gram-negative bacteria, comprising the step of propagating gram-negative bacteria according to the invention as described further below which contain genetic information for expressing LIp, intimin and said polypeptide of interest and expressing said LIp protein, intimin and polypeptide of interest.

The method according to the invention for presenting polypeptides allows a genotype-phenotype coupling, i.e. a correlation of the polypeptide of interest presented on the surface of the cell (the phenotype) and the genetic information contained in the corresponding cell encoding the polypeptide of interest (genotype). This coupling can easily be achieved by making sure that single cell clones are grown in physical/spatial separation, i.e. the clones are physically/spatially separated from each other during growth and lysis. By this measure it is ensured that the polypeptide of interest expressed in a specific clone is only presented on cells which also carry the corresponding genotype.
The term "clone" is an accepted term in the concerned technical field. It commonly relates to cells which originate from a common ancestor, i.e. a common mother cell and which are preferably genetically identical with respect to their relevant genetic characteristics within the range of normal spontaneously occurring genetic variation.

The physically/spatially separated growth of the cells in a method according to the invention can be achieved by methods well known in the art. It is, e.g., possible to grow the cell clones in separate physical compartments, preferably microcompartments.
A physical compartment may be any cavity or hole in a solid support such as a well in a microtiter plate or any container or vessel, such as an Eppendorf tube. Alternatively, the compartment, preferably the microcompartment, may also be a sphere or bead surrounding or providing a space in which the cells can be grown but from which the cells cannot escape. Preferably such microcompartments are semipermeable microcompartments. Semipermeable in this context means that nutrients and metabolites are able to diffuse through the material forming the microcompartment and that macromolecular complexes, such as proteins and cells cannot leave the microcompartment or enter into it.

An example for such semipermeable microcompartments are alignate beads, i.e. microcompartments composed of the biopolymer alignate.
The outer shell of such alignate microbeads is rigid enough to withstand the mechanical forces exerted during incubation in a flask or fermenter, while the material in the inside of the bead is of a gel-like nature and allows for replication of the cells.
Alginate is a polysaccharide extracted from algae, composed of the subunits β-D-mannuronic acid (M) and α-L-guluronic acid (G). The monomers form blocks that are aligned along the polysaccharide chain, where heteropolymeric blocks (MG-blocks) are alternating with homopolymeric blocks (M-blocks or G-blocks) (Smidsrod and
Haug 1972). The composition of the alginates is quite variable and depends on the organism and tissue from which they are extracted (Smidsrod and Skjak-Braek 1990). The block structure and composition of the alginate determines its functional properties. Owing to the caboxylate group at each subunit, the polymer possesses a strong negative charge. Intermolecular and intramolecular cross-linking can be mediated by divalent cations such as Ca²⁺, Zn²⁺ and Cu²⁺. The cation binding of alginates is dependent on their composition, as the cations preferentially bind to the cavitities formed by diaxially connected G-blocks. The binding can occur between G-blocks on the same or on neighbouring molecules, leading to a structure termed the egg box model. The length of the G-blocks within the alginate is thus the determining factor for alginate stability.
Alginate is frequently used as a matrix polymer for microencapsulation of pharmaceuticals, proteins, DNA and cells (Wan, Heng *et al.* 1993; Wan, Heng *et al.* 1994; Chan, Heng *et al.* 1997; Fundueanu, Esposito *et al.* 1998; Wee and Gombotz 1998). The microcompartments can be produced by emulsification technique (Heng, Chan *et al.* 2003). For this purpose, an aqueos solution containing the cells to be entrapped within the alginate beads is dispersed in an organic phase, so that a water-in-oil emulsion is formed. The formation of an emulsion is assisted by adding detergents. Afterwards, a calcium chloride solution is added to the organic phase and a co-emulsion is formed. If a droplet containing the alginate solution collides with a droplet containing the calcium chloride solution, the alginate solidifies (Heng, Chan *et al.* 2003). In this example, an organic phase consisting of iso-octane containing 3.8% sorbitane trioleate (Span85) and 0.25% polyoxyethylene-20-sorbitane (Tween85) was used.

In order to produce an emulsion of alginate beads in an organic phase, mechanical energy has to be supplied since the formation of the beads is thermodynamically unfavoured. The energy is supplied by stirring the solution with an impeller. The size of the produced alginate beads is determined by the speed of the impeller rotation, the physical properties of the impeller head, the viscosity of the alginate solution and the concentration of the calcium chloride solution. Heng *et al.* produced alginate microbeads with a diameter of 30 µm by using an emulsion of 2.5 % alginate in iso-octane/Span 85 and Tween 85 and a 6 % calcium chloride solution (Heng, Chan *et al.* 2003).

The biopolymer is not evenly distributed throughout the microbead. Rather, the alginate concentration is maximal at the edges of the bead and gets lower in the inside. The reason for this inhomogenity is unclear, but it is suspected that a simultanous diffusion of the calcium chloride to the inside and a diffusion of the polymer to the outside is responsible for this phenomenon (Smidsrod and Skjak-Braek 1990). Thus, beads with a relatively rigid outer shell and a liquid-like internal lumen are formed and provide good conditions for growth of bacteria inside the lumen of the beads. Furthermore, these beads have been characterized to be stable enough to allow for an incubation in liquid media with the necessary agitation.

The pore size of the alginate microcompartments is dependent on the fraction of G-blocks. The higher the fraction of the G-blocks, the higher the pores sizes of the microcompartments. Alginates with a high GC-content are inpermeable for molecules of a molecular weight larger than 30,000. The fusion protein claimed in this invention has a molecular weight of 36,000 even without the passenger protein. Therefore, the fusion protein can diffuse only very slowly, if at all, out of the alginate beads.
After growth of cells, the alginate microbeads can be dissolved by adding the calcium chelating agents EDTA or EGTA in a concentration of 20 mM in order to release the cells for further analysis or other purposes such as their use in screening methods. Thus, after dissolution of the beads, the cells can be subjected to further analyis. Beforehand, the alginate microbeads are preferably separated from the cells that grew outside the beads in the liquid medium. This can, e.g., be achieved by centrifugation of the bead/cell mixture through 20 % ficoll, in which the beads pellet faster since they have a higher density.

The method according to the invention for presenting a polypeptide on the surface of a cell is particularly suitable for use in screening methods aimed at identifying clones expressing polypeptide variants with desired characteristics and the corresponding nucleic acid sequences encoding them. As explained above, the method according to the present invention has the advantage that also polypeptides can be presented on the outer surface of cells which cannot pass the cell membrane, or, in the case of gram-negative bacteria, the periplasmic space and/or outer membrane, due to the partially lysing the cells.

In a screening method, cells can be used which encode a multitude of variants of a polypeptide of interest, i.e. a library of cell clones is prepared which express different variants or different types of a polypeptide of interest. Such a library of cell clones can be prepared according to methods well-known in the art, e.g. by transforming the cells to be employed in a method according to the invention with a library, such as a DNA library, encoding the polypeptide of interest variants. The polypeptides of interest are characterized in that they can bind on the surface of said cells. The rest of the polypeptide may vary and may be any type of polypeptide of interest. Such DNA libraries encoding a multitude of different polypeptides can be prepared according to methods well-known in the art, e.g. by in vitro or in vivo mutagenesis of a given nucleotide sequence. Methods for in vitro mutagenesis are, e.g. described in detail in US 20030036092. They comprise, e.g. the chemical mutagenesis, in particular of isolated DNA, gene amplification by error prone PCR and oligonucleotide mutagenesis. In vivo mutagenesis is also described in the literature and may be effected, e.g., by using mutagenizing agents or the use of E. coli mutator strains. Other possibilities for preparing corresponding libraries are the use of the ligation of randomized gene regions, exon shuffling and the like.

Thus, the present invention also relates to a method of screening for identifying cell clones which express a polypeptide having a desired property comprising the steps of
(a) propagating individual clones of cells, which contain genetic information for expressing a polypeptide of interest comprising a part which allows it to bind to the surface of said cells, in spatial separation and partially lysing the cells of the resulting population; and
(b) identifying those clones which present on their surface a polypeptide of interest with the desired property.
   Step (a) can be carried out according to methods well-known in the art and as described above.
   The identification in step (b) depends on the desired property. If, e.g., an enzyme with a certain activity is looked for, the identification step (b) may be an enzymatic assay. In case of a protein with a desired binding activity, the identification step (b) may, e.g., be a binding assay involving the ligand and, for example, a corresponding ligand-specific labelled antibody.
   The present invention also relates to a method for identifying a nucleotide sequence encoding a polypeptide with a desired property and the encoded polypeptide comprising steps (a) and (b) of the above-described screening method and further comprising the step of
(c) isolating the nucleotide sequence encoding the polypeptide of interest from a clone identified in step (b) and determining its nucleotide sequence and the encoded amino acid sequence.

The isolation of the nucleotide sequence encoding the polypeptide of interest can be carried out by methods well-known to a person skilled in the art and comprise, e.g., preparation of genomic or plasmid DNA from the identified cell clone (depending on whether the nucleotide sequence is located in the genome or on a plasmid) or PCR. Sequencing can be carried out according to methods known in the art.
With respect to the cells, the polypeptide of interest, the partially lysing etc. the same applies as has been set forth above in connection with the method according to the invention for presenting a polypeptide on the surface of cells.

The present invention also relates to the use of a partially lysed population of cells wherein the cells contain genetic information for expressing a polypeptide of interest which comprises a part which allows it to bind to the surface of said cells in any of the methods according to the invention as described above.

In another aspect the present invention relates to a cell of a gram-negative bacterium characterized in that it contains genetic information for expressing the following polypeptides:
(i) the outer membrane protein LIp; and
(ii) an intimin.

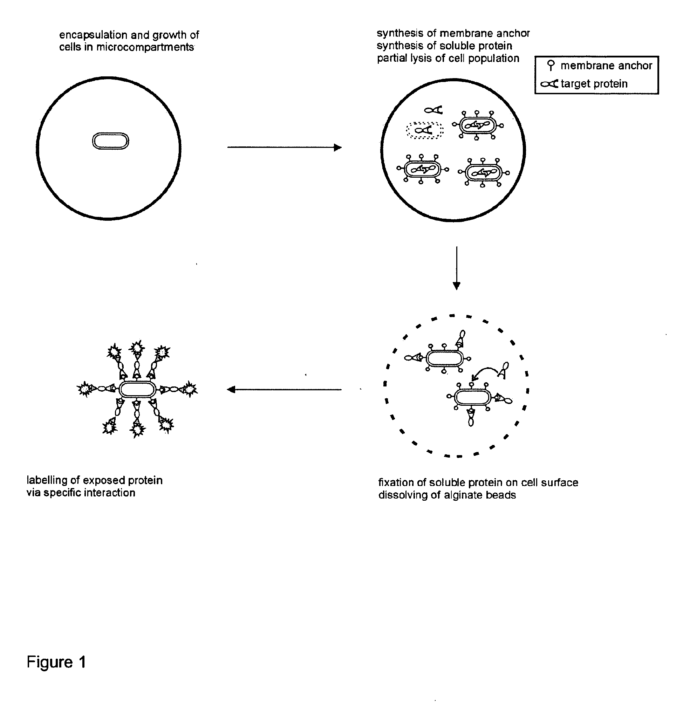

It has been found that the coexpression of the outer membrane protein LIp and of intimin in gram-negative bacteria has the effect that the bacterial cells become physically unstable such that a fraction, but not all, of the cells get lysed and release the cellular content. Thus, gram-negative bacterial cells coexpressing LIp and intimin can be used in the above described methods according to the invention for presenting polypeptides on the surface of cells, in particular gram-negative bacteria, and in the screening methods for identifying cell clones and nucleotide sequences. An example of this system using the above-described gram-negative bacterial cells is schematically represented in Figure 1 and can be summarized as follows: A gram-negative bacterium is provided which coexpresses LIp and intimin. The intimin in this specific example is furthermore fused to a domain capable of heterodimerization with a domain fused to the protein of interest. The gram-negative bacterium furthermore expresses a protein of interest containing a domain capable of heterodimerization with the heterodimerization domain fused to the intimin. When such a gram-negative bacterium cell is cultivated, a fraction of the resulting cells, but not all, will lyse. The still intact cells carry on their surface the intimin fused to the heterodimerization domain. Due to the lysis of a fraction of the cells, the protein of interest produced in these cells will be set free and released into the exterior. This protein of interest can then be bound to the still intact cells by the dimerization of the heterodimerization domains present on the intimin and present on the protein of interest. Thus, as a result, the protein of interest set free by the fraction of the lysed cells is bound (in this example) by protein-protein interaction to the surface of the still intact cells and is, thus, presented and displayed on the surface of the cells without the need to pass any cell membranes thereby avoiding the above-described disadvantages of the prior art.

It has been found that coexpression of LIp and intimin resulted in a partial lysis of a cell population of gram-negative bacterial cells coexpressing these proteins. In this respect, the important aspect is that not all but only a fraction of the bacterial cells is lysed. This allows to use such cells in a method for presenting proteins on the cell surface which are not or cannot be transported over the cell membranes.
It is evident to the person skilled in the art that the means and methods for ensuring binding of the protein of interest on the remaining intact cells are not restricted to the specific system as illustrated in Figure 1, but can be designed in many other possible ways. Thus, it is not mandatory that the protein of interest is bound to the cell surface of the cell by interaction with the intimin fusion construct. All other possible means and methods for binding a protein to a cell surface can be employed and some examples will be described further below.

The term "gram-negative bacterium" when used in the context of the present invention includes all possible gram-negative bacteria. Preferably, the bacterium belongs to an order selected from the group consisting of Neisseriales, Sphingomonadales, Rhizobiales, Enterobacteriales and Xanthomonadales. More preferably it belongs to a family selected from the group consisting of Neisseriaceae, Alcaligenaceae, Sphingomonadaceae, Rhizobiaceae, Enterobacteriaceae and
Xanthomonadaceae. Even more preferably, the bacterium belongs to a genus selected from the group consisting of Neisseria, Alcaligenes, Bordetella, Zymomonas, Agrobacterium, Salmonella, Shigella and Xanthomonas.
In a further preferred embodiment the gram-negative bacterium belongs to the genus Pseudomonas, more preferably to the species P. fluorescens, P. putida, P. alcaligenes or P. oleovorans.

In a particularly preferred embodiment the gram-negative bacterium is of the genus Escherichia and more preferably of the species E. coli.

The term "contains genetic information for expressing" means that the bacterium contains a nucleotide sequence, typically in the form of DNA, which encodes the corresponding polypeptide and which is under the control of regulatory elements allowing expression of the polypeptide in bacterial cells, in particular gram-negative bacterial cells. Such regulatory elements are exhaustively described in the literature and include in particular promoters, e.g. bacterial or viral promoters. It is possible to use promoters ensuring constitutive expression of the gene or inducible promoters which permit a deliberate control of the expression of the gene. Bacterial and viral promoter sequences possessing these properties are described in detail in the literature. Promoters permitting a particularly high expression of a downstream sequence are for instance the T7 promoter (Studier et al., Methods in Enzymology 185 (1990), 60-89), lacUV5, trp, trp-lacUV5 (DeBoer et al., in Rodriguez and Chamberlin (Eds.), Promoters, Structure and Function; Praeger, New York (1982), 462-481; DeBoer et al., Proc. Natl. Acad. Sci. USA (1983), 21-25), Ip1, rac (Boros et al., Gene 42 (1986), 97-100). Inducible promoters can also be used.

Preferred promoters are the lac promoter, the tetracycline promoter or the LIp promoter. In a particularly preferred embodiment the LIp is expressed from its own promoter and the intimin gene is under the control of the tetracycline promoter/operator sequence.

The genetic information may be present in the bacterial cells in any suitable form, e.g. integrated into the genome or present on an extrachromosomal DNA molecule, such as a plasmid. Expression vectors for expression of polypeptides in bacteria, in particular gram-negative bacteria, have been widely described in the literature. Generally, they contain a selection marker gene and an origin of replication ensuring replication in the host cell selected for expression. Moreover, they contain the coding sequence to be expressed flanked by regulatory regions. Examples for suitable plasmids for expressing intimin or LIp in the bacterial cells are the low-copy plasmid pTH19ks5 (Hashimoto-Gotoh, Yamaguchi et al. 2000), which contains a variant of the pSC101 replication origin with increased temperature-sensitivity, and the high-copy plasmid pHKREI (Kolmar, Ferrando et al., 1992).

The term "outer membrane protein Llp" relates to the LIp protein encoded and expressed by phage T5. This protein is encoded by the genome of the phage and integrates into the membrane of the host cells, in particular gram-negative bacteria, such as E. coli. It inactivates the bacterial receptor FhuA and prevents multiple infection of cells with the phages (Decker, Krauel et al. 1994). The LIp protein is described in the literature, e.g. in Decker, Krauel et al. (1994) and in Braun V., H. Killmann et al. (1994). The LIp protein expressed by the bacterial cells of the present invention is preferably the LIp protein having the amino acid sequence as disclosed in GenBank Accession number CAA53520 or in Decker, Krauel (1994). The amino acid sequence is also shown in SEQ ID NO: 2.

However, it is also possible to express an LIp protein in a gram-negative bacterium according to the present invention which has an amino acid sequence which differs from the above-mentioned LIp at one or more positions but which still shows the biological activities of LIp, i.e. it integrates into the membrane of host cells, in particular gram-negative bacterial cells, such as E. coli, and it inactivates the bacterial receptor FhuA. Thus, the LIp protein may also be an LIp protein which has at least 70% sequence identity, preferably at least 80% sequence identity, even more preferably at least 90% and particularly preferred at least 95% sequence identity to the above-cited amino acid sequence and which has the property that it leads to partial lysis of a population of gram-negative bacteria when expressed together with intimin. The nucleotide sequence encoding LIp present in the bacterial cell of the present invention is preferably a sequence comprising the coding region for LIp as disclosed in GenBank Accession number X7592 or Decker, Krauel et al. (1994). This sequence is also shown in SEQ ID NO: 1. However, it may also be a nucleotide sequence which is at least 60%, preferably at least 70%, more preferably at least 80%, even more preferably at least 90% and particularly preferred at least 95% identical to the above-mentioned nucleotide sequence and which encodes an LIp protein with the above-mentioned biological activities.

The term "intimin" refers to transmembrane proteins of the bacterial surface which play an essential role in the adhesion of enteropathogenic (EPEC) or enterohaemorrhagic E. coli bacteria (EHEC) (Luie et al., Infect. Immun. 61 (1993), 4085-4092). Pathogenic gram-negative bacteria have developed a number of secretion mechanisms during evolution in order to present on their surface binding proteins which interact with complementary receptors on the target cell (Klemm and Schembri, 2000b). Among these, the intimins belong to a family of bacterial adhesins which can bind specifically to receptors on eukaryotic cells (Vallance and Finlay, 2000). Enteropathogenic (EPEC) and Enterohaemorrhagic E. coli (EHEC) produce so called "attaching and effacing lesions" of the intestinal mucosa of the host organism (Moon, Whipp et al., 1983). The development of these lesions is initiated by the binding of the EHEC protein intimin to the Translocated Intimin Receptor (TIR), which is integrated in the cell membrane of the host cells (Kenny, De-Vinney, 1997). In the literature at least five different sub-types of intimin are described (Adu-Bobie, Frankel et al., 1998; Oswald, Schmidt et al., 2000). They are integrated into the outer membrane of E. coli cells via the N-terminus while the 280 C-terminal amino acids are exposed on the surface (Frankel, Candy et al., 1994).
As described above, intimins are found in E. coli cells, in particular enteropathogenic and enterohaemorrhagic E. coli cells. Different types of intimins have been found in different serotypes. Sequence comparisons between the intimins of EPEC and EHEC O157:H7 showed a considerable high degree of conservation between the genes (Yu and Kaper, 1992). The overall sequence identity is 83%, with the diversity being highest in the C-terminal region. The first three quarters of the protein, i.e. the 704 N-terminal residues are 94% identical. The last quarter only shows a sequence identity of 25% (Yu and Kaper, 1992). The sequence homologies on the nucleotide level are comparable to those on the amino acid level. The highly divergent C-terminal part of the protein represents the part which is responsible for receptor binding (Frankel, Candy et al., 1994). The different C-terminal sequences can be correlated with the different patterns of colonizations of the various strains. So far, at least five different variants of intimin are known which are designated α, β, γ, δ (Adu-Bobie, Frankel et al., 1998) and ε (Oswald et al., 2000), respectively. They mainly differ in the C-terminal domain in which the cell binding activity is located.

In principle, any intimin can be expressed in a gram-negative bacterial cell according to the invention. Preferably, it is one of the intimins disclosed in German patent application DE-A1 100 53224, in particular those disclosed on pages 17 to 43. More preferably it is an intimin of an EHEC bacterium and most preferably the intimin of the bacterium EHEC O157:H7, e.g. of the isolate deposited as ATCC 51658. The nucleotide sequence of this intimin gene (i.e. gene eaeA) and the corresponding amino acid sequence is available under GenBank Accession Number Z11541 and is published in Yu and Kaper (1992). The sequences are also shown as SEQ ID NOs: 3 and 4, respectively. However, it is also possible to express an intimin in the bacterial cells of the present invention which differs at one or more amino acid positions from any of the above-mentioned intimins. Thus, the intimin may also be an intimin which shows at least 60%, preferably at least 70%, more preferably at least 80%, even more preferably at least 90% and particularly preferred at least 95% sequence identity to any one of the above-mentioned intimins and which has the property that it leads to partial lysis of a population of gram-negative bacteria when coexpressed together with a LIp protein in these bacteria. Similarly, the nucleotide sequence encoding the intimin present in the bacterial cells of the present invention may be a nucleotide sequence as disclosed in DE-A1 100 53224 on pages 17 to 43**,** in GenBank Accession Number Z11541 or in SEQ ID NO: 3 or it may be a nucleotide sequence which is at least 60%, preferably at least 70%, more preferably at least 80%, even more preferably at least 90% and particularly preferred at laest 95% identical to a nucleotide sequence encoding an intimin as mentioned above.
In the context of the present invention the term "intimin" also includes truncated versions of an intimin protein, preferably an intimin protein which is truncated at its C-terminus. The cell-binding activity of intimins resides in two immunoglobulin-like domains and a lectin-like domain (Kelly et al., Nat. Struct. Biol. 6 (1999), 313-318) which are responsible for the binding to eukaryotic cells. These domains are located on the extracellular side.
The structure of the carboxyterminal domains has been determined by X-ray crystallographic analysis and NMR. The complete extracellular region forms an elongated structure comprising three immunoglobulin-like domains and one lectin-like domain (Kelly et al., loc. cit; Batchelor et al., 2000; Luo et al., 2000). The most C-terminal domain, which is responsible for receptor binding, is thus placed on a rather rigid arm which is connected to the transmembrane domain via a rather flexible hinge formed by two glycin residues (Luo et al., 2000).
It is assumed that the amino terminal part of intimin forms a porin-like structure built up of antiparallel β-sheets (Schirmer, 1998).

The specific intimin eaeA of EHEC O157:H7 used in the Examples of the present invention comprises 939 amino acids. The 280 carboxy-terminal amino acid residues are thought to be involved in receptor binding.
For the purpose of the present invention, i.e. to obtain lysis of a fraction of the gram-negative bacterial cells, the presence of the domains responsible for receptor binding is not necessary and, as shown in the Examples, lysis can be achieved when a truncated version of intimin is used which lacks the domain responsible for the adhesion to eukaryotic cells. Since this adhesion is part of the pathogenic nature of intimin expressing bacteria, it is preferred to use in the context of the present invention a truncated intimin which lacks at least one, more preferably at least two and most preferably at least all three of the above-mentioned extracellular domains responsible for adhesion to eukaryotic cells, i.e. the three C-terminal domains.
In a more preferred embodiment the intimin expressed by the bacterial cell according to the invention is a truncated intimin which comprises only the membrane anchoring domain. Optionally it may comprise additionally also one or more of the extracellular domains or parts thereof. In a particularly preferred embodiment the truncated intimin is an intimin as disclosed in DE-A1 10053224.
Even more preferably, the truncated intimin is a truncated version of the intimin encoded by the eaeA gene (GenBank Accession Number Z11541) of EHEC O157:H7 comprising only the N-terminal 659 amino acids encoded by codons 1 to 659 of the eaeA gene.

The intimin may, in a further aspect of the present invention, be coupled or fused to a domain or tag which allows a protein-protein interaction with a protein of interest to be expressed in the bacterial cell of the present invention. This aspect will be described in more detail further below.

The nucleotide sequences encoding the LIp protein and the intimin may be present on the same molecule or on separate molecules. For example, the intimin and LIp encoding sequences may be present on the same plasmid (as described in the attached Examples) or they may be present on separate plasmids. In principle, the two genes, if present on the same molecule, can be transcribed from a common promoter. However, it is preferable that transcription of each gene is under the control of its own promoter.

As described above, the cells according to the invention, e.g. the gram-negative bacterial cells expressing LIp and intimin, are particularly suitable for providing a system for presenting polypeptides of interest on the surface of cells, e.g. gram-negative bacteria, in particular such polypeptides which do not or cannot pass through the cytoplasmic cell membrane, the periplasm and/or the outer membrane. Thus, in a further aspect the present invention relates to an above-described gram-negative bacterial cell which furthermore contains genetic information for expressing a polypeptide of interest comprising a part (tag) which allows the polypeptide (when set free from lysed cells) to bind to the surface of intact gram-negative bacterial cells of the invention, i.e. to structures on the outer membrane.
With respect to the expression "contains genetic information for expressing" the same applies as has been set forth above in connection with the intimin and the LIp protein.
The term "a polypeptide of interest" refers to any possible protein considered to be expressed in the cell, i.e. this term can be used interchangeably with the general term "polypeptide". The phrase "of interest" should only specify that the polypeptide is of interest, e.g. for assessing whether it has certain properties or for using it in screening assays or the like.
The polypeptide of interest can in principle be any type of polypeptide, in particular of any origin, e.g. of viral, bacterial, fungal, plant or animal origin. It can be, e.g., a naturally occurring polypeptide or a variant thereof or it can be a mutated form. It can, of course, also be the product of expression of nucleotide sequences, e.g. DNA or RNA sequences, obtained by randomization processes which are, for example, used in in vitro and/or in vivo methods for molecular evolution. Preferably, the polypeptide of interest is a soluble protein. The term "soluble" in this context means that it is a protein which is synthesized in the cytosol of a cell, e.g. a bacterium, and which does not or cannot pass the cell membrane(s), e.g. the bacterial cytoplasmic membrane and/or the outer membrane, i.e. it does not or cannot reach the exterior if the cell is not lysed. Thus, it may be a protein which stays in the cytosol or it may be a protein which can pass the inner cytoplasmic bacterial membrane but not the outer membrane. In a preferred embodiment "soluble" means that the protein stays in the cytosol of the cell and does not or cannot pass the (bacterial) cytoplasmic membrane. Even more preferably, "soluble" means that the protein is not anchored in a membrane. As mentioned above, the soluble protein may be any type of soluble protein, e.g. an enzyme or part thereof, an antibody or antibody fragment, a binding protein, soluble domains from receptor molecules, in particular those involved in ligand binding, a chaperon, a structural protein, a regulatory protein, a signal transduction protein, a soluble domain of a membrane protein, etc. The soluble protein may also be a di- or multimeric protein. Both homo- and heterodimeric proteins are conceivable.

The term "comprising a part (tag) which allows the polypeptide, when set free from lysed cells, to bind to the surface of neighboring intact gram-negative bacterial cells of the invention" means that the polypeptide comprises a structure which allows it to bind to the surface of the bacterial cells according to the invention when it is set free from lysed cells, e.g. by interacting with structures on the outer membrane of the bacterial cells. This results in the display of the polypeptide of interest on the surface of the not lysed bacterial cells and, thus, the possibility to correlate the phenotype presented on the outside of these cells with the corresponding genotype carried by the cells.
The "part" which allows the soluble protein to be bound to the surface of a gram-negative bacterium according to the invention will also be referred to in the following as a "tag". A tag in this context means any possible type of physical entity which by way of interaction can confer the capacity of binding to the surface of a gram-negative bacterium according to the invention. The interaction may be a covalent or a non-covalent interaction. Preferably, the tag is a polypeptide and the interaction is by way of non-covalent interaction of the polypeptide with a proteinaceous or non-proteinaceous structure (e.g. a lipid, polysaccharide or lipopolysaccharide (LPS)) on the surface of the bacterium. Most preferably, the interaction is a protein-protein interaction, e.g. by way of receptor-ligand binding or by way of dimerization or oligomerization, preferably heterodimerization or -oligomerization.
It is evident for those skilled in the art that the binding of the polypeptide of interest on the surface of the gram-negative bacterium according to the invention can be achieved in various ways. In one aspect, it is, e.g. possible to design the polypeptide of interest in such a way, e.g. as a fusion protein, that it contains a tag which is capable of binding to a structure naturally found on gram-negative bacteria. For example, said tag may be an antibody or antibody fragment which recognizes a structure naturally existing on gram-negative bacteria. Such a structure naturally occurring on gram-negative bacteria are, e.g. membrane proteins (such as ompA), or lipopolysaccharide.
Alternatively, said part of the polypeptide of interest may be a polypeptide other than an antibody which specifically binds to a structure occurring on the surface of gram-negative bacteria. Examples of such polypeptides are polypeptides expressed by eukaryotic cells which bind to structures on the surface of bacterial cells. These include proteins which bind lipopolysaccharide, i.e. Lipopolysaccharide-Binding Protein (LBP), Bactericidal/Permeability-increasing Protein (BPI) or fragments thereof (Weiss, 2003). The eukaryotic protein CD14 is also capable of binding to lipopolysaccharide (Fenton, Golenbock, 1998) as well as Phospholipid Transfer Protein (PLTP) (Hailman et al., 1996).
Another possibility to ensure binding of the polypeptide of interest on the surface of gram-negative bacteria is to equip the soluble polypeptide with a tag which is capable of binding to a polypeptide or part thereof which is present on the outer surface of the outer membrane of the gram-negative bacteria and wherein said latter polypeptide or part thereof does naturally not occur on the surface of gram-negative bacteria. In particular, said latter polypeptide or part thereof is heterologous with respect to the gram-negative bacterium. Thus, it is possible to ensure binding of the soluble protein by making use of protein-protein interaction with another heterologous protein presented on the outer membrane of the gram-negative bacterium. One possibility in this regard is to equip the soluble protein with a tag which is capable of interacting with a heterologous structure presented on the outer membrane of the bacterium according to the invention, wherein said heterologous structure is fused to the intimin. Thus, in a preferred embodiment the present invention relates to a gram-negative bacterium which contains genetic information for expressing LIp, intimin and a polypeptide of interest, wherein the intimin comprises a tag and wherein the polypeptide of interest comprises a tag and wherein the tag of the intimin and the tag of the polypeptide of interest are capable of interacting with each other, i.e. to bind to each other. In a preferred embodiment the tags on the intimin and on the polypeptide of interest are of proteinaceous nature, i.e. built up of amino acids, and the interaction between the two tags is based on protein-protein interaction. More preferably, the tags on the intimin and on the polypeptide of interest, respectively, are polypeptide domains capable of dimerization, preferably of heterodimerization. Examples for systems allowing for such protein-protein interactions are (a) the use of antibodies (or fragments thereof with antigen binding capacity) and the corresponding antigen, (b) the use of leucine zipper domains, e.g. Fos and Jun (Kouzarides and Ziff, 1989), (c) receptor-ligand interactions, e.g. fibrinogen and α_{IIb}β_{III} integrin (Shattil et al., 1998), and (d) other protein-protein interactions, e.g. the binding of calmodulin by the molecule myk (a short peptide from the light chain of myokinase; Ikura et al., 1992).

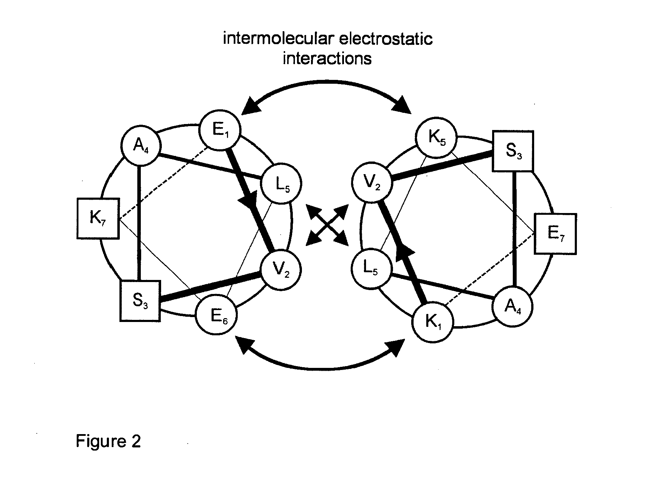

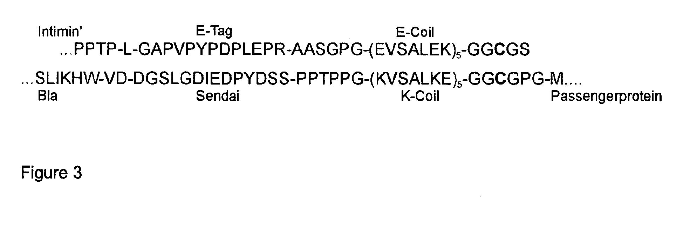

In a more preferred embodiment the tags present on the intimin and on the polypeptide of the present invention, respectively, are an E-coil and a K-coil, respectively. Such an E-coil and K-coil are capable of forming a heterodimer by specific protein-protein interaction. The structure and principle of interaction of the E-coil and the K-coil are described in the prior art, e.g. in Tripet, Yu et al. (1996) and in WO 97/12988 and particular reference is made to this document with respect to the way how exactly the E-coil and K-coil can be designed. Thus, the definition and possible designs of the E-coil and K-coil used in the present invention are preferably those as disclosed in WO 97/12988. In brief, the E- and K-coils are alphahelices one of which is rich in glutamate (i.e. the E-coil) and one of which is rich in lysine (i.e. the K-coil). The interaction of these two helices is based on two properties. First, there is electrostatic attraction between the negatively charged glutamate and the positively charged lysine. Secondly, there is hydrophobic interaction between the amino acids leucine and valin, which can be found in both of the helices. In order to prevent homodimerization, amino acids were choosen that are not too hydrophic, so that the electrostatic interaction becomes the determining force favouring a heterodimerization (Irvin, Hodges *et al.* 1996). The scheme of the interaction of these two helices is depicted in Figure 2. The binding of these two helices is characterized by a dissociation constant K_{d} between 0.3 and 1.3 nM with a high on and low off-rate (Tripet, Yu *et al.* 1996). Both helices can be arranged in a parallel as well as antiparallel fashion, where the parallel arrangement is slightly preferred (Irvin, Hodges *et al.* 1996). Each helix is followed by a cysteine residue at a distance of three amino acids. If the parallel dimer is formed, these cysteine residues are positioned close to each other and can be oxidized to form a covalent linkage of the passenger to the cell surface via disulphide bridge formation.
In a particularly preferred embodiment the E-coil and K-coil, respectively, have the amino acid sequences as depicted in Figures 2 and 3, i.e. the E-coil has the sequence (EVSALEK)₅ (SEQ ID NO: 5) and the K-coil has the sequence (KVSALKE)₅ (SEQ ID NO: 6). However, it is not absolutely necessary that five repetitions of the E- and K-coil are used. At least two, three or four repetitions should be used preferably.
As will be evident to the person skilled in the art, the location of the E-coil and the K-coil on the intimin or the polypeptide is not decisive. This means that the E-coil may be fused to the intimin (as described in the Examples) and the K-coil may be fused to the polypeptide of interest or, vice versa, the E-coil may be fused to the polypeptide of interest and the K-coil may be fused to the intimin.
By using the above approach, i.e. equipping the intimin and the polypeptide expressed by the gram-negative bacteria of the invention with tags which are able of specifically interacting with each other, it is possible to ensure that the polypeptide of interest expressed by the bacteria is bound to the surface of intact bacteria of the invention when it is released from lysed cells.
As will be evident for one skilled in the art, it is of course not only possible to have the above described tag presented on the cell surface by intimin, but the tag described in connection with intimin can, of course, also be presented by any other polypeptide which is expressed by the bacterium and which is exposed on the cell surface. Thus, the present invention also relates to a gram-negative bacterium containing genetic information for expressing LIp, intimin, a polypeptide of interest and a polypeptide exposed on the outer membrane of the gram-negative bacterium, wherein said polypeptide of interest and said polypeptide exposed on the outer membrane of the bacterium each comprise a tag and wherein the tag comprised in the polypeptide of interest is capable of specifically binding to the tag comprised in the polypeptide exposed on the outer membrane of the bacterium. With respect to the tags, the same applies as has been described above in connection with the embodiment in which the polypeptide of interest and the intimin comprise a tag. The polypeptide which is expressed by the gram-negative bacterium of the invention and which is exposed on the outer membrane of the bacterium may be any possible protein which can be expressed by gram-negative bacteria and exposed on the outer membrane. Such a protein is characterized in that it can pass the bacterial cytoplasmic membrane, the periplasmic space and the outer membrane and by that it is exposed on the outer cell membrane, e.g. by linkage or binding to a structure on the outer membrane or, preferably by being anchored in the outer membrane and displaying the tag on the external surface. Proteins which fulfill these criteria are known in the prior art and include, beside the above-mentioned intimin, for example porins (Lang 2000), autotransporter (Klauser, Pohlner et al. 1992), fimbriae (Klemm and Schembri 2000a), ice-nucleation-protein (Jung, Lebeault et al. 1998) and the fusion of the Lpp' and the ompA protein which is a fusion of the first nine amino acids of the lipoprotein Lpp of the outer membrane of E. coli and a truncated version of ompA (Francisco et al., 1992). The LIp protein, the intimin, the polypeptide of interest and/or the polypeptide exposed on the surface of the bacterium which carries a tag which interacts with a tag on the polypeptide of interest may optionally comprise further polypeptide domains, for example, amino acid sequences which allow detection of the presence of the above-mentioned polypeptides on the surface (i.e. on the outer membrane) of the bacteria according to the invention or which facilitate purification. An example of such amino acid sequences are epitopes which can be recognized by antibodies specific for such epitopes. Suitable epitopes are well-known in the art and include, e.g., the epitope recognized by the commercially available Anti-E antibody (Pharmacia, Uppsala, Sweden), i.e. the E-tag. The amino acid sequence of this epitope is shown in Figure 3 and has the amino acid sequence:

### GAPVPYPDPLEPR (SEQ ID NO: 7)

Another example is the epitope formed by the 13 C-terminal residues of Sendai virus L-protein having the following amino acid sequence:

### DGSLGDIEPYDSS (SEQ ID NO: 8)

(see also Einberger, Mertz et al. 1990). This epitope is recognized by the monoclonal anti Sendai antibody (VII-E-7).
A further Example is also the E2-tag, i.e. SSTSSDFRDR (SEQ ID NO: 9).
Another suitable marker sequence is the HA tag which corresponds to an epitope derived from the influenza hemagglutinin polypeptide (Wilson, Cell 37 (1984), 767). Another possible amino acid sequence which may be fused to the above-mentioned polypeptides is a hexa-histidine peptide, such as the tag provided in a pQE vector (Qiagen, Inc.) which provides, inter alia for convenient purification. Moreover, also glutathione-S-transferase (GST) may be fused to any of the above-mentioned polypeptides. It is an efficient detection and purification tag and enhances polypeptide stability.

The polypeptide of interest may also comprise additional sequences, such as, e.g. sequences of a carrier protein. The term "carrier protein" in this context is meant to refer to a polypeptide which allows for high level expression in gram-negative bacteria and/or which facilitates purification of a polypeptide containing it. Any carrier protein may be used in this respect. Possible examples are maltose-binding protein (MBP), glutathione-S-transferase (GST) and ProteinA. In a preferred embodiment the carrier protein is β-lactamase (Bla).

The different functional units of the above-described polypeptides are connected to each other by means well-known in the art. Preferably, they are linked by peptide bonds and more preferably they are separated by short linker sequences consisting of short stretches of amino acids, i.e. spacers. Examples for such spacers are depicted in Figure 3.

In another aspect the present invention relates to the use of nucleotide sequences encoding intimin and of nucleotide sequences encoding LIp protein for the preparation of gram-negative bacteria which, upon growth, are partly physically unstable. The term "partly physically unstable" means that upon growth a part of the cells of the bacterial population gets lysed whereas a part of the bacterial cells of the population remains intact. A "part" with respect to the lysed cells preferably means that at least about 5%, more preferably at least 10%, even more preferably at least 20%, particularly preferred at least 30%, in another preferred embodiment at least 40% and most preferred at least 50% of the cells in the population get lysed. A "part" furthermore means that not more than 95%, preferably not more than 90%, more preferably not more than 80%, even more preferably not more than 70%, particularly preferred not more than 60% and most preferably not more than 50% of the cells in the population get lysed.
More preferably, the present invention relates to the use of nucleotide sequences encoding LIp protein for the preparation of gram-negative bacterial cells which coexpress intimin and LIp. With respect to the terms "intimin" and "Llp" and with respect to the possibility of expressing these proteins in gram-negative bacteria the same applies as has been set forth above in connection with the bacteria according to the invention. Thus, nucleotide sequences encoding intimin and LIp protein, respectively, can be used to provide bacteria which, upon growth, result in a population of bacterial cells a part of which lyses and a part of which remains intact. Thus, the present invention also relates to a method for providing a bacterial culture comprising lysed cells and comprising intact cells, comprising the step of propagating a bacterial gram-negative cell according to the invention.

The present invention also relates to the use of a cell according to the invention which expresses said polypeptide of interest in a method according to the invention as described above.
As explained above, the coexpression of intimin and LIp protein in the bacteria leads to the lysis of a fraction of the propagated cells. However, some cells of the population remain intact. The lysis of a part of the cells allows the polypeptide of interest to locate to the extracellular space without the necessity to pass the outer membrane. The polypeptide of interest released from the lysed cells is then captured by the remaining intact cells on their surface. This is so because the polypeptide of interest comprises a part which allows the polypeptide to bind to the surface (i.e. outer membrane) of the gram-negative bacteria of the invention.
- **Figure 1**: shows a schematic representation of the method of the present invention for presenting and optionally also detecting a protein of interest ("target protein; soluble protein") on bacterial cells. "Membrane anchor" refers to the intimin coupled to a domain capable of heterodimerization.
- **Figure 2**: shows a schematic representation of the interaction of E-coil (left) and K-coil (right), which relies on the electrostatic attaction between glutamate (E1, E6) and lysine (K1, K5), and hydrophobic interactions between leucine (L5) and valine (V2). Only one out of five windings is shown.
- **Figure 3**: shows the sequence context of Intimin'-E-Tag-E-Coil (top) and Bla-Sendai-K-Coil-Passenger fusion proteins (bottom).
- **Figure 4**: shows a schematic representation of interactions of the E-coil/K-coil mediated interaction of the membrane anchor with the soluble protein.
- **Figure 5**: shows a schematic representation of the plasmids pBAIT and pPREY.
(A) the genetic elements on pBAIT: intimin', truncated intimin from EHEC O157:H7 ranging from codon 1 to 659; e-tag, E-tag-epitope encoding sequence; e-coill, coding sequence for the synthetic α-helix E-coil; pSC101, replication origin; kanR(agp5), kanamycin-resistance; Iacl, gene for lac-repressor; IIp, coding region for IIp lipoprotein, TetP/O, tetracycline-promotor/operator sequence.
(B) the genetic elements on pPREY: cat, chloramphenicol acetyl transferase gene; colE1, ColE1 replication origin; f1, replication origin of filamentous phage f1; tetR, gene encoding tetracycline repressor; bla, coding region of β-lactamase; sendai, 13-amino acid epitope of Sendai-virus-L-protein; k-coil, coding sequence for the synthetic α-helix K-Coil;
passenger, gene encoding passenger protein.

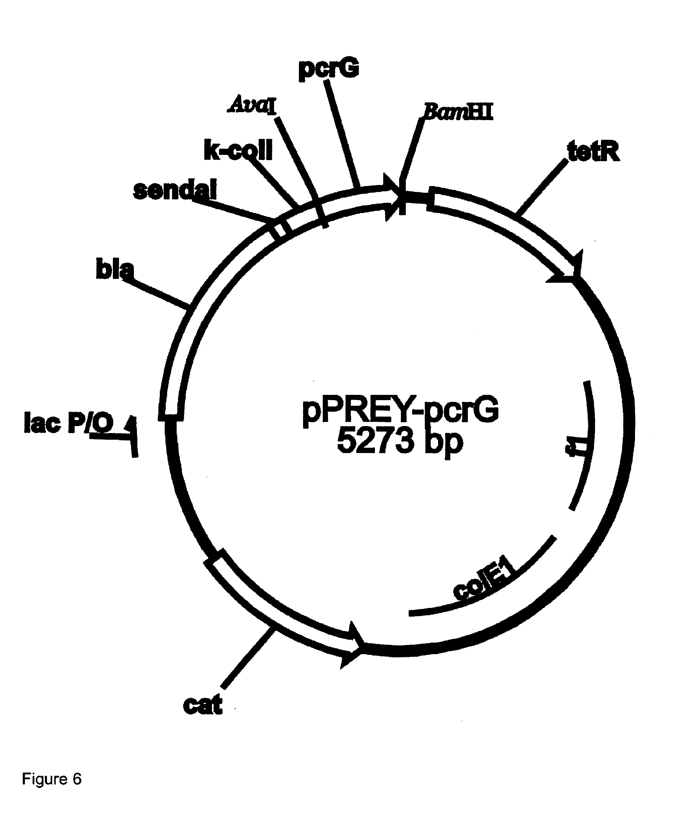

- **Figure 6**: shows the genetic elements on pPREY-pcrG: cat, chloramphenicol acetyl transferase gene; colE1, ColE1 replication origin; f1, replication origin of filamentous phage f1; tetR, gene encoding tetracycline repressor; bla, coding region of β-lactamase; sendai, 13-amino acid epitope of Sendai-virus-L-protein; k-coil, coding sequence for the synthetic α-helix K-Coil; pcrG, gene encoding PcrG.

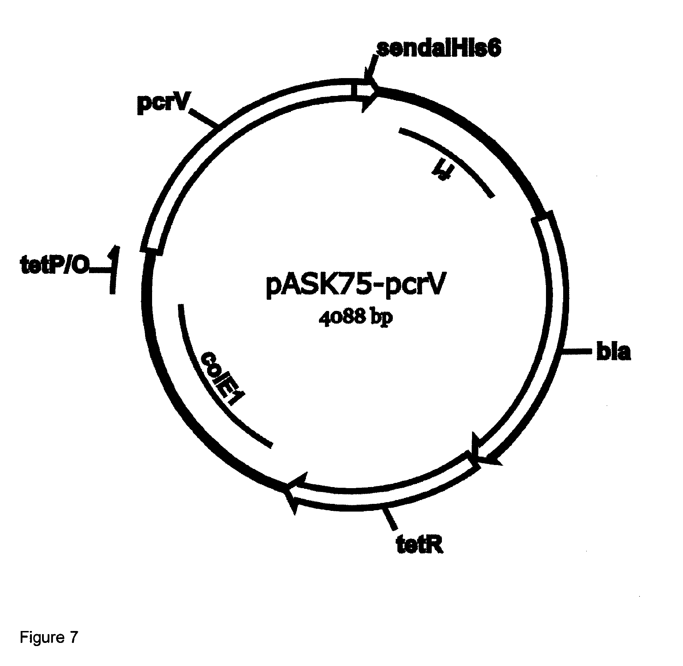

- **Figure 7**: shows the genetic elements on pASK75-pcrV: bla, coding region of β-lactamase; colE1, ColE1 replication origin; f1, replication origin of filamentous phage f1; tetR, gene encoding tetracycline repressor; sendHis6, 13-amino acid epitope of Sendai-virus-L-protein fused to a hexahistidine tag; k-coil, coding sequence for the synthetic α-helix K-Coil; pcrV, gene encoding PcrV.

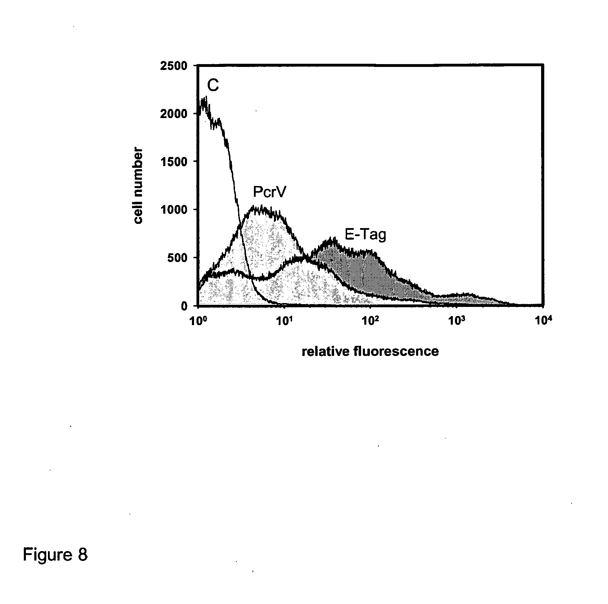

- **Figure 8**: shows the FACS analysis of induced 71-18mutS cells. C: control cells harbouring plasmid pBAIT, stained with biotinylated PcrV. PcrV: cells harbouring plasmids pBAIT und pPREY-pcrG, stained with biotinylated PcrV and streptavidin, R-phycoerythrin conjugate. E-Tag: cells harbouring plasmids PBAIT und pPREY-pcrG, stained with anti-E-Tag antibody.

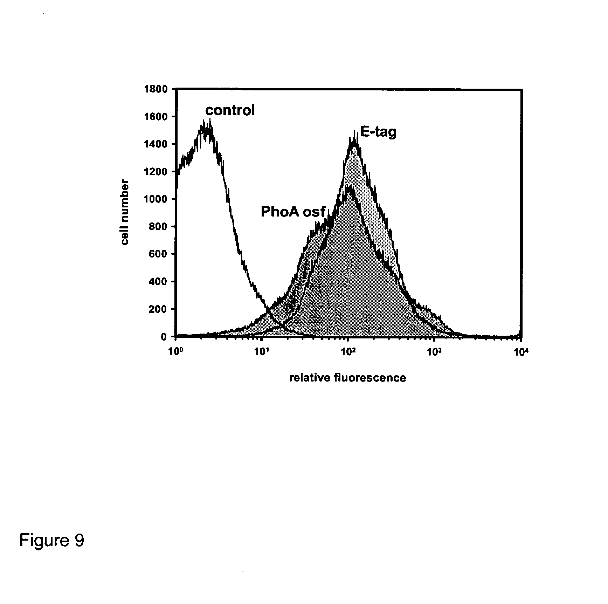

- **Figure 9**: shows the FACS analysis of fluorescently labeled E-coil presenting cells. Control: cells expressing the E-coil fusion stained with anti-PhoA antibody. E-tag: cells expressing the E-coil fusion stained with anti-E antibody. PhoA osf: cells expressing the E-coil fusion stained with osmotic shock fluid (osf) of cells expressing the K-coil-phoA fusion, then stained with anti-PhoA antibody.

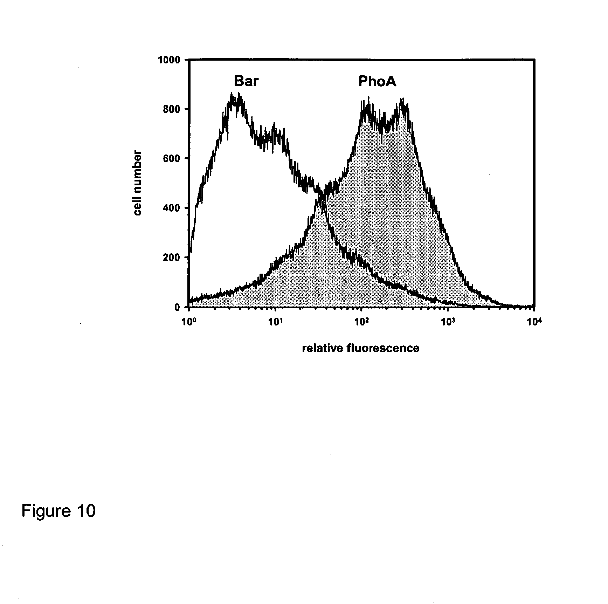

- **Figure 10**: FACS analysis of cells incubated with anti-PhoA antiserum, biotinylated anti-rabbit antibody and streptavidin, R-phycoerythrin. Bar: cells displaying an inactive form of the RNase barnase as negative control, PhoA: cells displaying alkaline phosphatase via ALD.

The following Examples serve to illustrate the invention.

### Example 1: Fixation of the soluble protein of interest to the surface of E. coli through a leucine zipper heterodimer

The fixation of the soluble protein of interest to the cell surface of the cell containing the respective gene was achieved by a specific protein-protein interaction, where one of the interaction partners is exposed on the cell surface while the other interaction partner is fused to the protein of interest on the genetic level. The surface-exposed interaction partner is fixed to the cell surface by fusion to a truncated intimin (intimin') (Wentzel, Christmann et al. 2001). Briefly, the intimin'-based cell surface display relies on the usage of a heterologous truncated intimin from enterohemorrhagic E. coli (EHEC), where the outermost three domains have been clipped off and replaced by the surface exposed interaction partner, whose surface exposition can be checked by staining against a specific epitope (German patent application DE-A1 10053224).
The interaction between the soluble protein of interest and the surface exposed intimin is performed by a synthetic heterodimer, consisting of two alpha helices, each of which is composed of five repetitions of seven amino acids (Tripet, Yu et al. 1996). While one of the alphahelices is rich in glutamate, the prevalent amino acid in the other alpha helix is lysine, hence the terms E-coil and K-coil were coined (Irvin, Hodges et al. 1996). The interaction of these two helices is based on two properties. First, there is electrostatic attraction between the negatively charged glutamate and the positively charged lysine. Secondly, there is hydrophobic interaction between the amino acids leucine and valin, which can be found in both of the helices. In order to prevent homodimerization, amino acids were chosen that are not too hydrophic, so that the electrostatic interaction becomes the determining force favouring a heterodimerization (Irvin, Hodges et al. 1996). The scheme of the interaction of these two helices is depicted in Figure 2. The binding of these two helices is characterized by a dissociation constant K_{d} between 0.3 and 1.3 nM with a high on and low off-rate (Tripet, Yu et al. 1996). Both helices can be arranged in a parallel as well as antiparallel fashion, where the parallel arrangement is slightly preferred (Irvin, Hodges et al. 1996). Each helix is followed by a cysteine residue at a distance of three amino acids. If the parallel dimer is formed, these cysteine residues are positioned close to each other and can be oxidized to form a covalent linkage of the passenger to the cell surface via disulphide bridge formation.
The E-coil part of the interaction pair is located to the bacterial cell surface via fusion to the intimin translocator domain (Wentzel, Christmann et al. 2001). Additionally, the coding sequence of the epitope against the commercially available Anti-E antibody (Pharmacia, Uppsala, Sweden) is fused to the heterologous intimin-e-coil gene. Cells that are expressing this fusion protein can be labeled with the anti-E antibody and surface exposition of the E-coil protein can be checked.

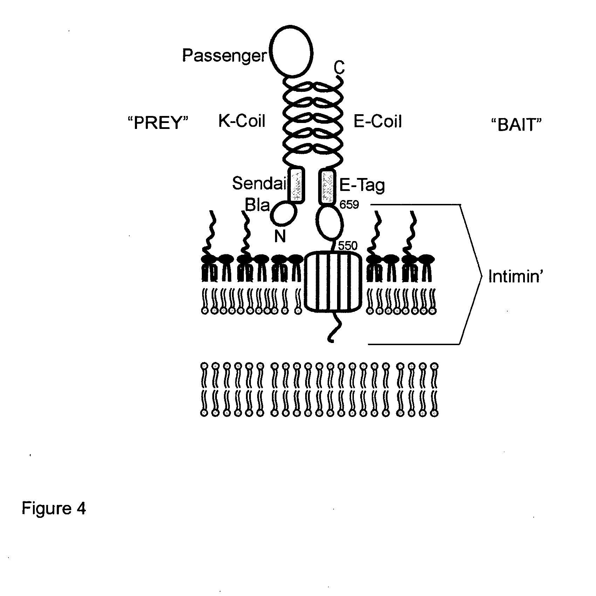

The soluble protein of interest, which is preceded in this example by the carrier protein β-lactamase (Bla) is fused to the K-coil helix and to an epitope against the Anti-Sendai antibody. This monoclonal anti-Sendai antibody (VII-E-7) is directed against a 13 residue C-terminal epitope of Sendai virus L-protein (DGSLGDIEPYDSS) (SEQ ID NO: 8) (Einberger, Mertz et al. 1990) and serves to detect the surface localization of the protein of interest. Those trained in the art will recognize that the soluble protein of interest can be fused to any carrier protein or can be used without a carrier protein, and that any (or no) epitope tag can be used. The functional units of the fusion proteins are separated by short stretches of amino acids, the spacers, as depicted in Figure 3. In Figure 4, it is shown how the fusion proteins are oriented towards the cell. The passenger-K-coil fusion is termed PREY, whereas the surface exposed E-coil constitutes the BAIT.

### Plasmids employed for production of heterologous proteins

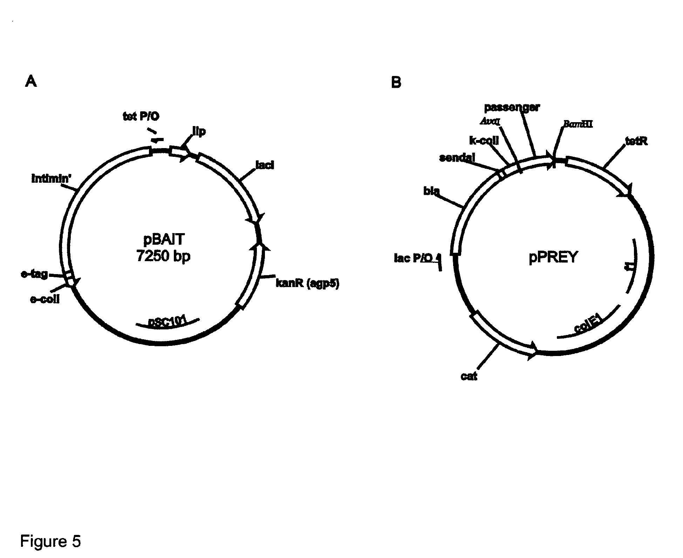

The cell surface exposed Intimin'-E-Coil fusion protein as well as the soluble PREY protein are encoded by the two plasmids pBAIT and pPREY, which will be described in the following section. Both plasmids are preferably used simultaneously.
The vector pBAIT (Figure 5A) is derived from the low-copy plasmid pTH19ks5 (Hashimoto-Gotoh, Yamaguchi et al. 2000). This plasmid contains a variant of the pSC101 replication origin, where a base pair exchange leads to an increased temperature-sensitivity. The propagation and segregation of this plasmid requires an incubation temperature of not more than 30 °C. The gene coding for Intimin'-E-tag-E-coil is under control of the tetracycline promoter (Skerra 1994). Furthermore, this plasmid contains the IIp-gene (Braun, Killmann et al. 1994) under control of its own promoter. The Iacl gene is also present on this plasmid and supplies the lac repressor in trans for the repression of the PREY-fusion protein on the other plasmid pPREY. The presence of the plasmid pBAIT enables the cells to grow in the presence of kanamycin.

The vector pPREY (Figure 5B) is a derivative of the high-copy plasmid pHKREI (Kolmar, Ferrando et al. 1992) containing a lac promoter to control the expression of the soluble fusion protein consisting of β-lactamase, an sendai-epitope sequence, K-coil and the passenger protein. The plasmid contains a colE1 replication origin and the chloramphenicol-acetyltransferase gene. Furthermore, pPREY contains the gene for the tetracycline repressor in order to control the expression of the intimin'-fusion on the plasmid PBAIT. The bacterial strain 71-18 mutS (Kramer, Kramer et al. 1984) is used as expression host, because this strain is resistant against the antibiotic tetracycline and thus allows for the usage of tetracycline in order to induce the expression of the intimin' fusion.

### Lysis of cells and growth in microcompartments

For the release of the soluble protein from inside the cells, the dissolution of a fraction of cells belonging to the same clonal population is necessary. The fraction of cells remaining intact must be high enough in order to allow for a selection procedure afterwards. In this example, the soluble protein of interest to be anchored to the cell surface is fused to β-lactamase, which allows for high level expression and facile purification of a fused passenger (Kolmar, Ferrando et al. 1992).
A partial lysis of the cells is achieved by synthesis of the LIp protein, which renders the cells resistant against an infection with the phage T5 (Braun, Killmann et al. 1994). LIp is encoded in the genome of the phage and integrates into the membrane of the host cells, inactivating the bacterial receptor FhuA and preventing multiple infection of cells with the phages (Decker, Krauel et al. 1994). The present inventors made the novel and unexpected finding that the expression of IIp in concert a truncated intimin, in this particular case with the intimin'-E-tag-E-Coil-protein of interest fusion, is sufficient to promote lysis of a fraction of the cell population.
During growth of the cells, the integrity of a clonal population should be ensured. This means that an individual protein of interest must only be fixed on the cells containing the genetic information for its own production. Cross interactions between cells of different genetic layout should be avoided. This can be achieved by spatial separation of the cells, e.g. by their entrapment in semipermeable microcompartments, e.g. composed of the biopolymer alginate. Whereas nutrients and metabolites are able to diffuse through the alginate microbead, macromolecular complexes such as proteins and cells cannot leave these compartments. The outer shell of the microbeads is rigid enough to withstand the mechanical forces exerted during incubation in a flask or fermenter, while the material in the inside of the bead is of a gel-like nature and allows for replication of the cells. Further properties of alginate and methods for producing alginate beads as well as their use to encapsidate microorganisms have been described herein-above.

### Example 2: Use of the system according to the invention to examine interaction between proteins

The principle usability of the autolysis display system described in the present invention was demonstrated by a model experiment, where the interaction of the proteins PcrG and PcrV from the pathogenic bacterium Pseudomonas aeruginosa was investigated.
In affinity-immunoblots, an interaction of PcrV with PcrG could be confirmed (Allmond, Karaca et al. 2003). Furthermore, it could be demonstrated that PcrG and PcrV form a stoichiometric 1:1 complex and have a very high affinity to each other with a dissociation constant k_{D} of 15.6 nM (Nanao, Ricard-Blum et al. 2003). Owing to its ability to form a high affinity complex to PcrV, it is well suited as a model to test the feasibility of the ALD system to investigate protein-protein interactions. This example makes use of the same surface localization, transmembrane translocator and microencapsulation techniques as described in Example 1.

### Experimental procedure

### Compositions of plasmids employed for this experiment

The plasmid pBAIT was the same as used in Example 1 and is shown in Figure 5A. Figure 6 shows the plasmid pPREY-pcrG, which is used for the production of the soluble β-Lactamase-Sendai-Kcoil-PcrG protein. Figure 7 shows the organization of the plasmid pASK75-pcrV, which is used for the production of the soluble PcrV protein

### Purification of PcrV

The PcrV protein was constructed with a C-terminal hexahistidine tag and was purified by metal chelate affinity chromatography as described (Christmann, Walter et al. 1999) and dialysed against PBS buffer.

### Packing of cells containing pBAIT and pPREY-pcrG into alginate microcompartments

The alginate solution was prepared as follows: 1.2 % (w/v) Protanal 20/40 F (FMI Biopolymer, Drammen, Norway) was dissolved in bidestilled water. The solution was filtered subsequently through filters with a pore size of 1.2 µm, 0.8 µm, 0.45 µm and 0.22 µm in order to remove contaminants such as proteins and polyphenols (Smidsrod and Skjak-Braek 1990).
71-18mutS cells were transformed with the plasmids pBAIT and pPREY-pcrG. A single clone was picked and inoculated in 50 ml dYT medium (1% yeast extract [w/v], 1.6% bacto tryptone [w/v], 0.5% NaCl [w/v]) containing 37.5 µg/ml kanamycin and 25 µg/ml chloramphenicol at 30°C over night. The next day, cells were subcultured in 50 ml dYT medium containing 37.5 µg/ml kanamycin and 25 µg/ml chloramphenicol at 30°C for four hours. Then, the optical density (OD₆₀₀) was adjusted in PBS to a value of 2.0. 2 ml of the cell suspension were mixed with 8 ml of the alginate solution and 21.6 ml of iso-octane, 3.8% (v/v) Span 85 in a 100 ml beaker. The solution was stirred with an impeller rotor (diameter 31 mm, slope 1.6, manufacturer Graupner, cat. No. 2327.21), at 1200 RPM for 15 min. Then, 1 ml of 8.5% (v/v) Tween 85 was added and the solution was stirred for further 15 min. Finally, a solution of 6% (w/v) calcium chloride was added and the solution was again stirred for 15 min. In order to separate the alginate microbeads from the organic solvent, the organic phase was layered onto 20 ml of water in a 50 ml falcon tube and centrifuged for 10 min at 20°C and 3000 RPM. The pellet containing the alginate beads was transferred into 100 ml dYT containing 37.5 µg/ml kanamycin and 25 µg/ml chloramphenicol and grown at 30°C for 2 h. Then, 0.2 µg/ml anhydrotetracyline were added to induce the expression from the tetracycline promoter, therefore stimulating the synthesis of the intimin' fusion protein. After 24 h of growth at room temperature, the expression of the soluble passenger protein was induced by adding 1 mM IPTG and the cells were grown over night.

### Extraction of bacterial cells from alginate beads

After incubation the medium was centrifuged for 15 min at 750 RPM and 20°C (Rotanta/RPC, Hettich, Germany). During this centrifugation step, most of the beads travel to the pellet, while a large part of the cells does not. The pellet was resuspended in 30 ml bidestilled water and centrifuged again under the same conditions. The pellet was resuspended in 2 ml bidestilled water. Each 1 ml was layered onto a cushion of 20% ficoll (w/v) (Pharmacia Fine Chemicals, Upsala, Sweden) and centrifuged for 15 min at 750 RPM and 4°C. The alginate microbeads travelled to the pellet, while the majority of the cells remained in the solution, therefore this step served to separate the free bacterial cells from the alginate beads. The beads were resuspended in 2 ml PBS containing 20 mM EDTA or EGTA and incubated for 20 min while shaking in order to dissolve the alginate beads. Then, the cells were pelleted by centrifugation (4000 RPM, 12 min, 20°C) and the procedure was repeated once more. The final pellet was dissolved in 500 µl PBS.

### Staining of cells with anti-E antibody

After addition of 1 µl of the Anti-E antibody (1 mg/ml), cells were incubated for 10 min at room temperature. After addition of 500 µl PBS, cells were centrifuged for 1 min and resuspended in 10 µl PBS containing biotinylated goat anti-mouse immunoglobulin (1:10 dilution). After 10 min incubation at room temperature and addition of 500 µl PBS, cells were pelleted as above and resuspended in 10 µl PBS containing streptavidin, R-phycoerythrin conjugate (1:10 dilution), followed by 10 min incubation at RT. Finally, after addition of 500 µl PBS, cells were pelleted and resuspended in 100 µl PBS for flow cytometry analysis. 300,000 events were collected on a Cytomation MoFlo cell sorter. Parameters were set as follows: forward scatter, side scatter: 730 (LIN mode, amplification factor 6); FL1 (FITC): 600 (LOG mode); FL2 (PE): 600 (LOG mode); trigger parameter: side scatter. The sample flow rate was adjusted to an event rate of approximately 30,000 s⁻¹.

### Biotinylation of PcrV

5 mg PcrV protein were incubated with a threefold molar excess of EZ-Link Sulfo-NHS-LC-LC-biotin (Pierce, Rockford, IL, USA, Cat. No. 21338) for 1h at room temperature. Then, the reaction solution was dialysed against 5 L PBS over night at 4°C. Biotinylation was verified using HABA/Avidin reagent (Sigma, Cat. No. H2153).

### Analysis of binding of PcrG to PcrV

Cells were incubated on ice in 1 µM of soluble biotinylated pcrV for 20 min. After addition of 500 µl PBS, cells were centrifuged for 1 min and resuspended in 10 µl PBS containing streptavidin, R-phycoerythrin conjugate (Molecular Probes, Cat. No. S866, 1:10 dilution), followed by 5 min incubation at RT. Finally, after addition of 500 µl PBS, cells were pelleted and resuspended in 100 µl PBS for flow cytometry analysis. 300,000 events were collected on a Cytomation MoFlo cell sorter. Parameters were set as follows: forward scatter, side scatter: 730 (LIN mode, amplification factor 6); FL1 (FITC): 600 (LOG mode); FL2 (PE): 600 (LOG mode); trigger parameter: side scatter. The sample flow rate was adjusted to an event rate of approximately 30,000 s⁻¹.

### Results

### Production and analysis of alginate beads

71-18mutS cells containing the plasmids pBAIT and pPREY-pcrG were packaged into alginate beads as described in the experimental procedures part. When working with a repertoire of bacterial clones, the number of cells may be in the range of up to 10¹⁰. Therefore, the number of beads produced must be in the same range in order to accommodate all bacterial clones. Furthermore, the size of the beads must be sufficient to allow for multiple rounds of growth of the bacteria inside those cells. In order to estimate the sizes of the alginate microbeads. The produced alginate microbeads range in diameter from 5 to 15 µm and thus have a volume between 500 and 14000 µm³. A bacterial cell has a volume of roughly 1 µm³ (Kubitschek and Friske 1986) and thus the alginate beads provide enough space for multiple rounds of cell division. The maximum number of beads of these sizes that can be formed from 8 ml of alginate suspension lies between 6 x 10⁸ and 1.6 10¹⁰. Therefore, this method seems suitable even to handle larger repertoires.
After growth and induction of cells as described above the mixture of beads and cells were separated by centrifugation in 20% (w/v) ficoll, where the alginate beads sediment earlier due to their higher density.

### Binding of surface exposed PcrG to soluble PcrV

The cells regained from the inside of the beads were stained with anti-E antibody and biotinylated PcrV, respectively, as described in the experimental procedures section. As a negative control, cells that only contained the plasmid pBAIT and were also grown inside alginate microcompartments, were stained with biotinylated PcrV. The stained cells were analysed by FACS (Figure 8).

As can be seen from the FACS histogram, cells expressing the soluble Bla-Sendai-K-Coil-PcrG could be stained with biotinylated PcrV, whereas cells containing only pBAIT could not. Therefore, it can be concluded that the binding of PcrV to the cell surface is a result of the surface localization of PcrG.
This shows that the autolysis system of the present invention is in principle suitable for the detection of protein-protein interactions.

### Example 3: Surface presentation of alkaline phosphatase (PhoA)

The exposition of active enzymes on the surface of Escherichia coli cells is of great interest for research and industry. A fair number of enzymes has been successfully displayed on the surface of microorganisms (Becker, Schmoldt et al. 2004). However, the display of functional enzymes with a high tendency to fold already in the cytoplasm or periplasm or that are only active as dimers or oligomers has been notoriously difficult. So far, it has not been possible to display the alkaline phosphatase (PhoA) from E. coli in an active form. It is only active as a homodimer (Stathopoulos, Georgiou et al. 1996) and contains 449 amino acids in each subunit (Holtz and Kantrowitz 1999).
This example describes the display of PhoA on the cell surface of E. coli cells and its detection using a polyclonal antiserum directed against PhoA (Rockland Immunochemicals). The autolysis system of the present invention thus represents a novum which can be used for the display of active multimeric enzymes on the surface of bacterial cells.
For the production of soluble PhoA protein, a variant of pPREY lacking the tetR gene was used. This variant is called pPREY2.

### Experimental procedures

### Fixation of phoA to the cell surface of E. coli and detection

E. coli cells of the strain KS474 (Strauch and Beckwith 1988) were transformed with the plasmid pPREY2-PhoA. A single clone was propagated in dYT containing 25 µg/ml chloramphenicol and the expression of the soluble fusion protein was induced by adding 1 mM IPTG at an OD₆₀₀ of 0.2. After 15 h of growth, the periplasmic protein fraction was isolated. The cells were pelleted by centrifugation and resuspended in 200 mM Tris-Cl, pH 9.0, 100 mM EDTA, 20% (w/v) sucrose and incubated on ice for 1 hour. Cells were pelleted by centrifugation and subjected to osmotic shock by resuspension in 10 mM Tris-Cl, pH 9.0. Bacterial fragments were removed by centrifugation at 13,000 x g for 30 min to yield cleared osmotic shock fluid.

E. coli cells of the strain 71-18mutS (Kramer, Kramer et al. 1984) were transformed with the plasmid pBAIT and a single colony was inoculated in dYT complexed with kanamycin. Expression of the fusion protein was induced by adding tetracycline. When the cells reached an OD₆₀₀ of 0.4, 300 µl of the cells were pelleted by centrifugation.
The cells producing the Intimin'-E-tag-E-Coil fusion protein were incubated with 25 µl of the cleared osmotic shock fluid from the cells expressing the soluble bla-k-coil-phoA for 10 min on ice. As a control, untransformed 71-18mutS cells were treated equally. After addition of 1 µl of the polyclonal anti-PhoA antiserum (1 mg/ml), cells were incubated for 10 min at room temperature. After addition of 500 µl PBS, cells were centrifuged for 1 min and resuspended in 10 µl PBS containing biotinylated goat anti-rabbit immunoglobulin (1:10 dilution). After 10 min incubation at room temperature and addition of 500 µl PBS, cells were pelleted as described above and resuspended in 10 µl PBS containing streptavidin, R-phycoerythrin conjugate (1:10 dilution), followed by 10 min incubation at RT. Finally, after addition of 500 µl PBS, cells were pelleted and resuspended in 100 µl PBS for flow cytometry analysis. 300,000 events were collected on a Cytomation MoFlo cell sorter. Parameters were set as follows: forward scatter, side scatter: 730 (LIN mode, amplification factor 6); FL1 (FITC): 600 (LOG mode); FL2 (PE): 600 (LOG mode); trigger parameter: side scatter. The sample flow rate was adjusted to an event rate of approximately 30,000 s⁻¹.

### Test of enzymatic activity of surface localized PhoA

In order to detect enzymatic activity of PhoA located on the surface of E. coli, cells presenting only the synthetic alpha-helix E-Coil were resuspended in 100 mM Tris-HCl, pH 9.0, 100 mM NaCl, 50 mM MgCl₂. The OD₆₀₀ of the cell suspension was adjusted to a value of 0.4 and the cells were incubated with 25 µl of the cleared osmotic shock fluid from the cells expressing the soluble bla-k-coil-phoA for 10 min on ice. 50 µl of a 2 M p-nitrophenylphosphate solution were added to the sample and the degradation of the substrate was followed photometrically at 410 nm in a spectrophotometer (Shimadzu UV-1601) against 100 mM Tris-HCl, pH 9.0, 100 mM NaCl, 50 mM MgCl₂ as a reference substance.

As a negative control, the same cells that were not incubated with the osmotic shock fluid were used.

### Packing of cells expressing soluble PhoA into alginate microcompartments and immunological detection of PhoA surface localization

71-18mutS cells were transformed with the plasmids pBAIT and pPREY2-PhoA. A single clone was picked and inoculated in 50 ml dYT medium (1% yeast extract [w/v], 1.6% bacto tryptone [w/v], 0.5% NaCl [w/v]) containing 37.5 µg/ml kanamycin and 25 µg/ml chloramphenicol at 30°C over night. The next day, cells were subcultured in 50 ml dYT medium containing 37.5 µg/ml kanamycin and 25 µg/ml chloramphenicol at 30°C for four hours. Then, the cells were packaged into alginate microbeads as described above. After growth, the cells were extracted from the beads as described above.
The surface localization of PhoA was verified by staining the cells with polyclonal anti-PhoA antiserum (1 mg/ml) as described above.

### Results

### Fixation of alkaline phosphatase on the surface of E-coil presenting E. coli cells

In order to investigate the general feasibility to anchor PhoA to the surface of E. coli cells via the K-Coil/E-Coil heterodimer, cells synthesizing the Intimin'-E-tag-E-coil fusion were incubated with osmotic shock fluid (osf) of cells expressing the bla-K-coil-phoA fusion as described in the experimental procedures section. The surface localization of the Intimin'-E-Tag-E-Coil fusion was verified by staining with the anti-E antibody. The binding of PhoA to the cell surface via the dimerization of K-Coil and E-Coil was demonstrated by staining with an anti-PhoA antibody, biotinylated second antibody and streptavidin, R-phycorerythrin conjugate. As a negative control, E-Coil presenting cells that were not incubated with the osmotic shock fluid were stained with anti-PhoA antibody, biotinylated second antibody and streptavidin, R-phycorerythrin conjugate. The fluorescence of the cells was determined by FACS (Figure 9). This experiment showed that the E-coil is located on the cell surface.

The enzymatic activity of the cells that were incubated with the osf was assayed by determining the turnover of the alkaline phophatase substrate p-nitrophenol as described in the experimental procedures part. As a negative control, cells that were not incubated with the osf were also assayed. It was determined that only cells that were treated with the osf show a degradation of the substrate.
This experiment demonstrated the surface localization of the alkaline phosphatase.

### Display of PhoA on cells grown in alginate microbeads

Bacterial cells of the strain 71-18mutS were transformed with the plasmids pBAIT and pPREY2-PhoA and packaged into alginate microbeads as described before. Cells that displayed a variant of the Bacillus amyloliquefaciens barnase (Schmoldt, Wentzel et al. 2005) via the ALD system were also packaged into beads and served as the negative control. The cells within the beads were incubated in dYT medium complexed with the respective antibiotics. After ficoll centrifugation and dissolution of the microspheres by adding EDTA as described, the cells were immunofluorescently labeled. As primary antibody, anti-PhoA antiserum was used. Biotinylated anti-rabbit antibody was employed as second antibody, and streptavidin, R-phycorerythin was used for fluorescence labelling. The cellular fluorescence was measured by FACS as shown in Figure 10.

Taken together, these results demonstrate that the autolysis system of the present invention can be employed to display an enzymatically active alkaline phosphatase on the cell surface of Escherichia coli.

### References

Adams, T. M. and H. Kolmar (2004). Enzyme Engineering by Microbial Cell Surface Display. Enzyme Functionality: Design, Engineering and Screening. A. Svendsen. New York, MArcel Dekker: 599-616.

Adams, T. M., A. Wentzel, et al. (2005). "Intimin-mediated export of passenger proteins requires maintenance of a translocation-competent conformation." J Bacteriol 187(2): 522-533.

Adu-Bobie, J., G. Frankel, et al. (1998). "Detection of intimins alpha, beta, gamma, and delta, four intimin derivatives expressed by attaching and effacing microbial pathogens." J Clin Microbiol 36(3): 662-668.

Allmond, L. R., T. J. Karaca, et al. (2003). "Protein binding between PcrG-PcrV and PcrH-PopB/PopD encoded by the pcrGVH-popBD operon of the Pseudomonas aeruginosa type III secretion system." Infect Immun 71(4): 2230-2233.

Assenmacher, M., S. Miltenyi, et al. (2003). Direct selection of antigen-specific T cells, compositions obtained thereby and methods of use thereof. USA, Miltenyi Biotech GmbH (DE).

Batchelor, M., S. Prasannan, et al. (2000). "Structural basis for recognition of the translocated intimin receptor (Tir) by intimin from enteropathogenic Escherichia coli." Embo J 19(11): 2452-2464.

Becker, S., H. U. Schmoldt, et al. (2004). "Ultra-high-throughput screening based on cell-surface display and fluorescence-activated cell sorting for the identification of novel biocatalysts." Curr Opin Biotechnol 15(4): 323-329.

Blocker, A., N. Jouihri, et al. (2001). "Structure and composition of the Shigella flexneri "needle complex", a part of its type III secreton." Mol Microbiol 39(3): 652-663.

Braun, V., H. Killmann, et al. (1994). "Inactivation of FhuA at the cell surface of Escherichia coli K-12 by a phage T5 lipoprotein at the periplasmic face of the outer membrane." J Bacteriol 176(15): 4710-4717.

Chan, L. W., P. W. S. Heng, et al. (1997). "Effect on cellulose derivatives on alginate microspheres prepared by emulsification." J Microencapsul 14: 545-555.

Christmann, A., K. Walter, et al. (1999). "The cystine knot of a squash-type protease inhibitor as a structural scaffold for Escherichia coli cell surface display of conformationally constrained peptides." Protein Eng 12(9): 797-806.

Cordes, F. S., K. Komoriya, et al. (2003). "Helical structure of the needle of the type III secretion system of Shigella flexneri." J Biol Chem 278(19): 17103-17107.

Decker, K., V. Krauel, et al. (1994). "Lytic conversion of Escherichia coli by bacteriophage T5: blocking of the FhuA receptor protein by a lipoprotein expressed early during infection." Mol Microbiol 12(2): 321-332.

Einberger, H., R. Mertz, et al. (1990). "Purification, renaturation, and reconstituted protein kinase activity of the Sendai virus large (L) protein: L protein phosphorylates the NP and P proteins in vitro." J Virol 64(9): 4274-4280.

Fenton MJ, Golenbock DT. "LPS-binding proteins and receptors". J Leukoc Biol. 1998 Jul;64(1):25-32.

Francisco, J.A., Earhart, C.F. and Georgiou, G., Transport and anchoring of β-lactamase to the external surface of Escherichia coli. Proc Natl Acad Sci U S A, 1992. 89(7): pages 2713-2717.

Frankel, G., D. C. Candy, et al. (1994). "Characterization of the C-terminal domains of intimin-like proteins of enteropathogenic and enterohemorrhagic Escherichia coli, Citrobacter freundii, and Hafnia alvei." Infect lmmun 62(5): 1835-1842.

Fundueanu, G., E. Esposito, et al. (1998). "Preparation and charaterization of Ca-alginate microspheres by a new emulsification method." Int J Pharm 170: 11-21.

Griffiths, A. D. and D. S. Tawfik (2003). "Directed evolution of an extremely fast phosphotriesterase by in vitro compartmentalization." Embo J 22(1): 24-35.

Hashimoto-Gotoh, T., M. Yamaguchi, et al. (2000). "A set of temperature sensitive-replication/-segregation and temperature resistant plasmid vectors with different copy numbers and in an isogenic background (chloramphenicol, kanamycin, IacZ, repA, par, polA)." Gene 241(1): 185-191.

Hailman E, Albers JJ, Wolfbauer G, Tu AY, Wright SD. Neutralization and transfer of lipopolysaccharide by phospholipid transfer protein. J Biol Chem. 1996 May 24;271(21):12172-12178.

Heng, P. W., L. W. Chan, et al. (2003). "Formation of alginate microspheres produced using emulsification technique." J Microencapsul 20(3): 401-413.

Holtz, K. M. and E. R. Kantrowitz (1999). "The mechanism of the alkaline phosphatase reaction: insights from NMR, crystallography and site-specific mutagenesis." FEBS Lett 462(1-2): 7-11.

Hueck, C. J. (1998). "Type III protein secretion systems in bacterial pathogens of animals and plants." Microbiol Mol Biol Rev 62(2): 379-433.

Ikura, M., Clore, G.M., Gronenborn, A.M., Zhu, G., Klee, C.B. and Bax, A., Solution structure of a calmodulin-target peptide complex by multidimensional NMR. Science, 1992. 256(5057): pages 632-638.

Irvin, R. T., R. S. Hodges, et al. (1996). Coiled-Coil Heterodimer Methods And Compositions For The Detection And Purification Of Expressed Proteins. Canada.

Jung, H. C., J. M. Lebeault, et al. (1998). "Surface display of Zymomonas mobilis levansucrase by using the ice-nucleation protein of Pseudomonas syringae." Nat Biotechnol 16(6): 576-580.

Kenny, B., R. DeVinney, et al. (1997). "Enteropathogenic E. coli (EPEC) transfers its receptor for intimate adherence into mammalian cells." Cell 91(4): 511-520.

Klauser, T., J. Pohlner, et al. (1992). "Selective extracellular release of cholera toxin B subunit by Escherichia coli: dissection of Neisseria Iga beta-mediated outer membrane transport." Embo J 11 (6): 2327-2335.

Klemm, P. and M. A. Schembri (2000a). "Fimbriae-assisted bacterial surface display of heterologous peptides." Int J Med Microbiol 290(3): 215-221.

Klemm, P. and M. A. Schembri (2000b). "Bacterial adhesins: function and structure." Int J Med Microbiol 290(1): 27-35.

Kolmar, H., E. Ferrando, et al. (1992). "General mutagenesis/gene expression procedure for the construction of variant immunoglobulin domains in Escherichia coli. Production of the Bence-Jones protein REIv via fusion to beta-lactamase." J Mol Biol 228(2): 359-365.

Kondo, A. and M. Ueda (2004). "Yeast cell-surface display--applications of molecular display." Appl Microbiol Biotechnol 64(1): 28-40.

Kouzarides T, Ziff E. Behind the Fos and Jun leucine zipper. Cancer Cells. 1989 Nov;1(3):71-76.

Kramer, B., W. Kramer, et al. (1984). "Different base/base mismatches are corrected with different efficiencies by the methyl-directed DNA mismatch-repair system of E. coli." Cell 38(3): 879-887.

Kubitschek, H. E. and J. A. Friske (1986). "Determination of bacterial cell volume with the Coulter Counter." J Bacteriol 168(3): 1466-1467.

Lang, H. (2000). "Outer membrane proteins as surface display systems." Int J Med Microbiol 290(7): 579-585.

Luo, Y., E. A. Frey, et al. (2000). "Crystal structure of enteropathogenic Escherichia coli intimin-receptor complex." Nature 405(6790): 1073-1077.

Matson, J. S. and M. L. Nilles (2001). "LcrG-LcrV interaction is required for control of Yops secretion in Yersinia pestis." J Bacteriol 183(17): 5082-5091.

Matson, J. S. and M. L. Nilles (2002). "Interaction of the Yersinia pestis type III regulatory proteins LcrG and LcrV occurs at a hydrophobic interface." BMC Microbiol 2(1): 16.

Moon, H. W., S. C. Whipp, et al. (1983). "Attaching and effacing activities of rabbit and human enteropathogenic Escherichia coli in pig and rabbit intestines." Infect Immun 41(3): 1340-1351.

Nanao, M., S. Ricard-Blum, et al. (2003). "Type III secretion proteins PcrV and PcrG from Pseudomonas aeruginosa form a 1:1 complex through high affinity interactions." BMC Microbiol 3(1): 21.

Oswald, E., H. Schmidt, et al. (2000). "Typing of intimin genes in human and animal enterohemorrhagic and enteropathogenic Escherichia coli: characterization of a new intimin variant." Infect Immun 68(1): 64-71.

Pedruzzi I, Rosenbusch JP, Locher KP. Inactivation in vitro of the Escherichia coli outer membrane protein FhuA by a phage T5-encoded lipoprotein. FEMS Microbiol Lett. 1998 Nov 1;168(1):119-125. Samuelson, P., E. Gunneriusson, et al. (2002). "Display of proteins on bacteria." J Biotechnol 96(2): 129-154.

Schaffitzel, C., J. Hanes, et al. (1999). "Ribosome display: an in vitro method for selection and evolution of antibodies from libraries." J Immunol Methods 231(1-2): 119-135.

Schirmer, T. (1998). "General and specific porins from bacterial outer membranes." J Struct Biol 121(2): 101-109.

Schmoldt, H. U., A. Wentzel, et al. (2005). "A fusion protein system for the recombinant production of short disulfide bond rich cystine knot peptides using barnase as a purification handle." Protein Expr Purif 39(1): 82-89.

Shattil, S. J., Kashiwagi, H. and Pampori, N. (1998). Integrin signaling: the platelet paradigm. Blood 91 (8), 2645-2657.

Skerra, A. (1994). "Use of the tetracycline promoter for the tightly regulated production of a murine antibody fragment in Escherichia coli." Gene 151(1-2): 131-135.

Smidsrod, O. and A. Haug (1972). "Dependence upon the gel-sol state of the ionexchange properties of alginates." Acta Chem Scand 26(5): 2063-74.

Smidsrod, O. and G. Skjak-Braek (1990). "Alginate as immobilization matrix for cells." Trends Biotechnol 8(3): 71-78.

Smith, G. P. (1985). "Filamentous fusion phage: novel expression vectors that display cloned antigens on the virion surface." Science 228(4705): 1315-1317.

Stathopoulos, C., G. Georgiou, et al. (1996). "Characterization of Escherichia coli expressing an Lpp'OmpA(46-159)- PhoA fusion protein localized in the outer membrane." Appl Microbiol Biotechnol 45(1-2): 112-119.

Strauch, K. L. and J. Beckwith (1988). "An Escherichia coli mutation preventing degradation of abnormal periplasmic proteins." Proc Natl Acad Sci U S A 85(5): 1576-1580.

Tripet, B., L. Yu, et al. (1996). "Engineering a de novo-designed coiled-coil heterodimerization domain off the rapid detection, purification and characterization of recombinantly expressed peptides and proteins." Protein Eng 9(11): 1029-4102.

Vallance, B. A. and B. B. Finlay (2000). "Exploitation of host cells by enteropathogenic Escherichia coli." Proc Natl Acad Sci U S A 97(16): 8799-8806.

Wan, L. S. C., P. W. S. Heng, et al. (1993). "Influence of hydrophile-lipophile balance on alginate microspheres." Int J Pharm 95: 77-83.

Wan, L. S. C., P. W. S. Heng, et al. (1994). "Surfactant effects on alginate microspheres." Int J Pharm 103: 267-275.

Wee, S. and W. R. Gombotz (1998). "Protein release from alginate matrices." Adv Drug Deliv Rev 31 (3): 267-285.

Weiss J. "Bactericidal/permeability-increasing protein (BPI) and lipopolysaccharide-binding protein (LBP): structure, function and regulation in host defence against Gram-negative bacteria". Biochem Soc Trans. 2003 Aug;31 (Pt 4):785-790.

Wentzel, A., A. Christmann, et al. (2001). "Display of Passenger Proteins on the Surface of Escherichia coli K-12 by the Enterohemorrhagic E. coli Intimin EaeA." J Bacteriol 183(24): 7273-7284.

Yu, J. und Kaper, J. B. (1992). Cloning and characterization of the eae gene of enterohaemorrhagic Escherichia coli O157:H7 Mol Microbiol 6 (3), 411-417.

## Claims

1. A method for presenting a polypeptide of interest on the surface of cells containing genetic information for expressing said polypeptide **characterized in that** said polypeptide comprises a part which allows it to bind to the surface of said cells and **in that** the cells are propagated and partially lysed during or after propagation.

2. The method of claim 1 **characterized in that** individual cell clones are propagated in spatial separation.

3. A method of screening for identifying cell clones which express a polypeptide having a desired property comprising the steps of
(a) propagating individual clones of cells, which contain genetic information for expressing a polypeptide of interest which comprises a part which allows it to bind to the surface of said cells, in spatial separation and partially lysing the cells of the resulting population; and
(b) identifying those clones which present on their surface a polypeptide of interest with the desired property.

4. A method for identifying a nucleotide sequence encoding a polypeptide with a desired property and the encoded polypeptide comprising the steps of
(a) propagating individual clones of cells, which contain genetic information for expressing a polypeptide of interest which comprises a part which allows it to bind to the surface of said cells, in spatial separation and partially lysing the cells of the resulting population; and
(b) identifying those clones which present on their surface a polypeptide of interest with the desired property; and
(c) isolating the nucleotide sequence encoding the polypeptide of interest from a clone identified in step (b) and determining its nucleotide sequence and the encoded amino acid sequence.

5. The method of any one of claims 2 to 4, wherein the individual cell clones are propagated in separate cells of microtiter plates.

6. The method of any one of claims 2 to 4, wherein the individual cell clones are propagated in semipermeable microcompartments.

7. The method of claim 6, wherein the semipermeable microcompartments are alginate beads.

8. The method of any one of claims 1 to 7, wherein said cells are eukaryotic cells.

9. The method of any one of claims 1 to 7, wherein said cells are prokaryotic cells.

10. The method of claim 9, wherein said prokaryotic cells are gram-negative bacterial cells.

11. The method of claim 10, wherein the gram-negative bacterial cells are **characterized in that** they contain genetic information for expressing the following polypeptides:
(i) the outer membrane protein LIp; and
(ii) an intimin.

12. The method of claim 11, wherein the LIp protein comprises the amino acid sequence as shown in SEQ ID NO: 2.

13. The method of claim 11 or 12, wherein the intimin is an intimin of an enterohaemorrhagic E. coli (EHEC).

14. The method of claim 13, wherein the intimin is the intimin of EHEC O157:H7.

15. The method of claim 14, wherein the intimin is the intimin encoded by the eaeA gene.

16. The method of any one of claims 11 to 15, wherein the intimin is truncated at the C-terminus.

17. The method of any one of claims 10 to 16, wherein said polypeptide of interest binds to a polypeptide, lipid, polysaccharide or lipopolysaccharide on the outer membrane of said gram-negative bacterial cell.

18. The method of any one of claims 11 to 17, wherein the intimin comprises a tag which is capable of interacting with said polypeptide of interest.

19. A cell of a gram-negative bacterium **characterized in that** it contains genetic information for expressing the following polypeptides:
(i) the outer membrane protein LIp; and
(ii) an intimin.

20. The cell of claim 19, wherein the LIp protein comprises the amino acid sequence as shown in SEQ ID NO: 2.

21. The cell of claims 19 or 20, wherein the intimin is an intimin of an enterohaemorrhagic E. coli (EHEC).

22. The cell of claim 21, wherein the intimin is the intimin of EHEC O157:H7.

23. The cell of claim 22, wherein the intimin is the intimin encoded by the eaeA gene.

24. The cell of any one of claims 19 to 23, wherein the intimin is truncated at the C-terminus.

25. The cell of any one of claims 19 to 24, further comprising genetic information for expressing a polypeptide of interest wherein said polypeptide of interest comprises a tag which allows said polypeptide to bind to the outer membrane of said cell.

26. The cell of claim 25, wherein said tag binds to a polypeptide, lipid, polysaccharide or lipopolysaccharide on the outer membrane of said cell.

27. The cell of claim 25, wherein the intimin comprises a tag which is capable of interacting with the tag comprised in the polypeptide of interest.

28. The cell of claim 27, wherein the tag comprised in the intimin and the tag comprised in the polypeptide of interest are polypeptide domains capable of heterodimerization.

29. The cell of claim 28, wherein one of the tags is an E-coil and wherein the other tag is a K-coil.

30. The cell of claim 25 further comprising genetic information for expressing a polypeptide exposed on the outer membrane of said cell wherein said polypeptide comprises a tag which is capable of interacting with the tag comprised in the polypeptide of interest.

31. Use of a nucleotide sequence which codes for LIp and of a nucleotide sequence which codes for intimin for the preparation of gram-negative bacterial cells which coexpress intimin and LIp.

32. A method for providing a bacterial cell culture of gram-negative bacteria comprising lysed cells and comprising intact cells, comprising the step of propagating a cell of any one of claims 19 to 30.

33. Use of a cell of any one of claims 25 to 30 in a method according to any one of claims 1 to 10.

34. Use of a partially lysed population of cells wherein the cells contain genetic information for expressing a polypeptide of interest which comprises a part which allows it to bind to the surface of said cells in a method of any one of claims 1 to 18.
